# EUROPEAN PATENT APPLICATION

(11) **EP 4 782 458 A1**
(43) Date of publication of application: **29.07.2026**
(21) Application number: 26154210.4
(22) Date of filing: 26.01.2026
(51) Int. Cl.: C07H 19/10, C07H 21/02

(54) **5 -PHOSPHATE MODIFIED NUCLEOSIDE, AND PREPARATION METHOD AND USE THEREOF**

(30) Priority: 27.01.2025 CN 202510125941
(71) Applicant: Beijing Youcare Kechuang Pharmaceutical Technology Co., Ltd., Daxing District, Beijing 100176 (CN); Hangzhou Tianlong Pharmaceutical Co., Ltd., Hangzhou, Zhejiang 310020 (CN)
(72) Inventor: HUANG, Zeao, Hangzhou, 310020 (CN); SONG, Gengshen, Hangzhou, 310020 (CN); WANG, Jie, Hangzhou, 310020 (CN); YANG, Shuo, Hangzhou, 310020 (CN); LI, Zhenmin, Hangzhou, 310020 (CN); YANG, Yang, Hangzhou, 310020 (CN); YU, Fei, Hangzhou, 310020 (CN); CHEN, Aifu, Hangzhou, 310020 (CN)
(74) Representative: Dehns

(57) **Abstract**

The present disclosure relates to the field of biomedicine, and specifically provides a 5'-phosphate modified nucleoside, and a preparation method and use thereof. The present disclosure provides a modified nucleoside compound, wherein the modified nucleoside compound is a compound represented by formula (I), a pharmaceutically acceptable salt thereof, or a stereoisomer thereof. The oligonucleotide comprising the modified nucleoside compound provided by the present disclosure exhibits significantly enhanced drug efficacy.

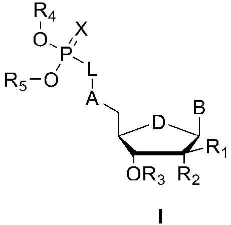

## Description

The present application claims the priority of Chinese patent application 202510125941.6 filed on January 27, 2025.

### TECHNICAL FIELD

The present disclosure relates to the field of biomedicine, specifically to a 5'-phosphate modified nucleoside, and a preparation method and use thereof.

### BACKGROUND

Small nucleic acid drugs achieve target selection through base complementary pairing and directly target specific genes, possessing high specificity, and expand drug targets to the upstream mRNA of pathogenic proteins, regulating the expression of target genes at the post-transcriptional level. Compared with antibody drugs and small molecule drugs, small nucleic acid drugs have advantages such as abundant targets, short research and development cycles, and long-lasting efficacy. There are many types of small nucleic acid drugs, mainly including an antisense oligonucleotide (ASO), a small interfering oligonucleotide (siRNA), a micro-oligonucleotide (miRNA), a nucleic acid aptamer (aptamer), and the like, wherein ASO, siRNA, and aptamer are the three types of small nucleic acid drugs that are most intensively studied.

siRNA drugs exert their effects through the RNA interference mechanism. The 5' end of the siRNA antisense strand that enters the cytoplasm via the endocytic pathway is phosphorylated by the intracellular kinase Clpl, forming a phosphate group at the 5' end of the antisense strand. The phosphate group at the 5' end can bind to the Ago2 protein, thereby promoting the entry of siRNA into the RNA-induced silencing complex (RISC). As the sense strand of siRNA is cleaved and removed, the resulting active RISC pairs and binds with the target mRNA, and the target mRNA is cleaved by the endonuclease within RISC, thereby reducing the expression of the target mRNA. The released active RISC species will continue to search for target mRNA for binding, enter the catalytic cycle, and continue to exert the efficacy. Therefore, the 5' end of the antisense strand in the double-stranded siRNA must carry a phosphate group in order to specifically bind to the side chain residues of the Ago2 domain, which is very important for the activity of RNA interference. However, studies have shown that after siRNA modified with a natural phosphate group at the 5' end enters the endocytic pathway, the acidic phosphatase in lysosomes rapidly degrades this terminal phosphate, which will limit their bioavailability *in vivo* (Reka AH, et al. Nucleic Acids Res., 2017, 45, 7581), thereby affecting the efficacy.

In some cases, replacing the natural phosphate group at the 5' end of the siRNA antisense strand with a phosphate ester analogue can improve the *in vivo* efficacy of siRNA, because the modified phosphate ester is not a substrate of phosphatases in the body, so after modification, it can resist degradation by exonucleases and enhance the *in vivo* silencing effect of siRNA. However, siRNA obtained by existing modification methods still suffers from problems such as insufficient resistance to exonuclease degradation, insignificant enhancement of the silencing effect of siRNA, or adverse impacts on the silencing effect of siRNA.

Therefore, it is desirable to develop modified nucleoside compounds that, after being introduced into siRNA, can improve the efficacy of siRNA drugs more effectively and stably.

### SUMMARY

In order to solve the above problems existing in the prior art, the present disclosure provides a 5'-phosphate modified nucleoside, and a preparation method and use thereof. Specifically, the present disclosure designs a modified nucleoside compound, which can be efficiently introduced into the 5' end of an oligonucleotide chain by a solid-phase synthesis method. Compared with the prior art, oligonucleotides comprising the modified nucleoside compounds of the present disclosure exhibit stronger inhibitory efficiency against target genes.

The inventors of the present disclosure unexpectedly found that introducing an alkyl group between the phosphorus atom of the phosphate and the 5'-hydroxyl group of the sugar ring to form nucleoside compounds can effectively enhance the resistance of siRNA and the like to nuclease degradation while improving the *in vivo* silencing effect of siRNA. By introducing nucleotides comprising such modified nucleoside compounds into an oligonucleotide, it is possible to obtain nucleic acid drugs with improved efficacy.

Based on this, the first aspect of the present disclosure provides a modified nucleoside compound, wherein the modified nucleoside compound is a compound represented by formula (I), a pharmaceutically acceptable salt thereof, or a stereoisomer thereof: 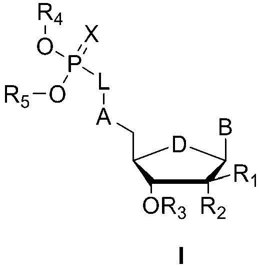 wherein
R₁ and R₂ are each independently selected from H, halogen, C₁₋₆ alkyl, and C₁₋₆ alkoxy;
R₃ is hydrogen, a protecting group, or a phosphorus-containing active reactive group;
R₄ and R₅ are each independently selected from hydrogen, C₁₋₆ alkyl, -CH₂CH₂CN, and - CH₂O(CO)C(CH₃)₃;
X is O or S;
L is C₁₋₆ alkyl, C₂₋₅ alkenyl, or C₂₋₅ alkynyl;
A is O or S;
B is H, a modified or unmodified base or a salt thereof;
D is O, S, or -CH₂-.

According to some preferred embodiments of the present disclosure, R₄ and R₅ are not simultaneously isopropyl.

According to some preferred embodiments of the present disclosure,
R₁ is H, and R₂ is -OCH₃;
R₄ and R₅ are each independently selected from -CH₂CH₃, -CH₃, and H;
L is CH₂.

According to some preferred embodiments of the present disclosure, R₁ is H.

According to some preferred embodiments of the present disclosure, R₂ is -OCH₃.

According to some preferred embodiments of the present disclosure, R₃ is a phosphorus-containing active reactive group.

According to some preferred embodiments of the present disclosure, R₄ is -CH₃ or -CH₂CH₃.

According to some preferred embodiments of the present disclosure, R₅ is -CH₃ or -CH₂CH₃.

According to some preferred embodiments of the present disclosure, X is O.

According to some preferred embodiments of the present disclosure, L is -CH₂- or -CH₂CH₂-.

According to some preferred embodiments of the present disclosure, A is O.

According to some preferred embodiments of the present disclosure, B is 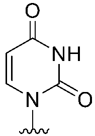 or 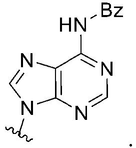 " 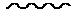 " indicates the connection position. "Bz" refers to benzoyl.

According to some preferred embodiments of the present disclosure, D is O.

According to some preferred embodiments of the present disclosure, the phosphorus-containing active reactive group is any one of a phosphoramidite, an H-phosphonate, a phosphotriester, or a phosphorus-containing chiral auxiliary.

According to some preferred embodiments of the present disclosure, R₃ is 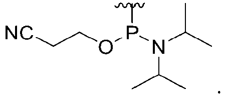 " 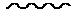 " indicates the connection position.

According to some preferred embodiments of the present disclosure, R₄ and R₅ are the same.

According to some particularly preferred embodiments of the present disclosure, the compound represented by formula (I) is any one or a combination of at least two of the following compounds: YK-VP-001, YK-VP-002, YK-VP-003, YK-VP-004, and YK-VP-005: 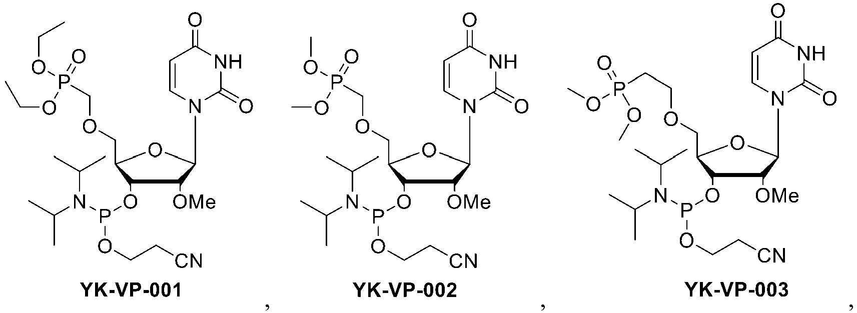 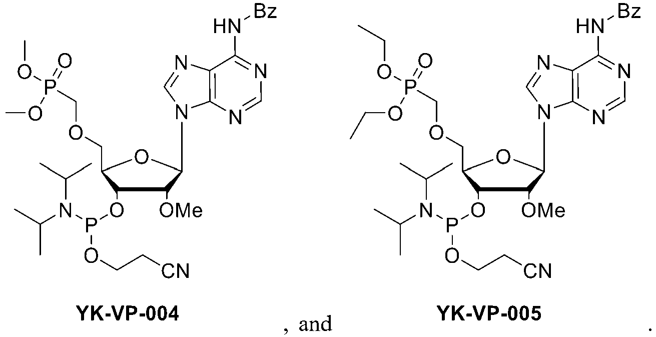 "Bz" refers to benzoyl, and "OMe" refers to methoxy.

Furthermore, the present disclosure also provides the use of the modified nucleoside compound according to the first aspect in improving the stability of oligonucleotides (e.g., improving resistance to the degradation effects by exonucleases).

Based on this, the present disclosure also provides a method of improving the stability of oligonucleotides (e.g., improving resistance to the degradation effects by exonucleases), the method comprising using a nucleotide comprising the modified nucleoside compound according to the first aspect of the present disclosure to replace at least part of the structural moieties (nucleotides) in an oligonucleotide.

Preferably, using a nucleotide comprising the modified nucleoside compound according to the first aspect of the present disclosure to replace 1-3 (most preferably 1) nucleotides at the 5' end of the oligonucleotide.

Furthermore, the present disclosure also provides the use of the modified nucleoside compound according to the first aspect in improving the *in vivo* function of oligonucleotides *(e.g.,* improving the silencing effect of siRNA).

Based on this, the present disclosure also provides a method of improving the *in vivo* function of oligonucleotides (*e.g*., improving the silencing effect of siRNA), the method comprising using a nucleotide comprising the modified nucleoside compound according to the first aspect of the present disclosure to replace at least part of the structural moieties (nucleotides) in an oligonucleotide.

Preferably, using a nucleotide comprising the modified nucleoside compound according to the first aspect of the present disclosure to replace 1-3 (most preferably 1) nucleotides at the 5' end of the oligonucleotide.

Furthermore, the present disclosure also provides the use of the modified nucleoside compound according to the first aspect in simultaneously improving the stability of oligonucleotides *(e.g.,* improving resistance to the degradation effects by exonucleases) and the *in vivo* function of oligonucleotides (*e.g*., improving the silencing effect of siRNA).

Based on this, the present disclosure also provides a method of simultaneously improving the stability of oligonucleotides (*e.g*., improving resistance to the degradation effects by exonucleases) and improving the *in vivo* function of oligonucleotides *(e.g.,* improving the silencing effect of siRNA), the method comprising using a nucleotide comprising the modified nucleoside compound according to the first aspect of the present disclosure to replace at least part of the structural moieties (nucleotides) in an oligonucleotide.

Preferably, using a nucleotide comprising the modified nucleoside compound according to the first aspect of the present disclosure to replace 1-3 (most preferably 1) nucleotides at the 5' end of the oligonucleotide.

The second aspect of the present disclosure provides an oligonucleotide, wherein the structural moiety of the oligonucleotide comprises a nucleotide comprising the modified nucleoside compound according to the first aspect. That is, the oligonucleotide provided by the present disclosure may be formed by replacing part of the structural moieties (nucleotides) in a conventional oligonucleotide with nucleotides synthesized from the modified nucleoside compound of the present disclosure *(i.e.,* the compound represented by formula (I) or a pharmaceutically acceptable salt thereof) and phosphoric acid.

In the oligonucleotide provided by the present disclosure, part of the structural moieties (nucleotides) may comprise the modified nucleoside compound provided in the first aspect, or all structural moieties may comprise this modified nucleoside compound. When part of the structural moieties comprise this modified nucleoside compound, the other part of the structural moieties in the oligonucleotide may be provided by conventional nucleotides or by nucleotides comprising other existing modified nucleoside compounds.

In the present disclosure, the structural moiety of an oligonucleotide refers to a nucleotide. As is well known in the art, a nucleotide is a compound composed of a base (purine base or pyrimidine base), a pentose sugar (ribose or deoxyribose), and a phosphate group. A base condenses with a pentose sugar to form a nucleoside, which further reacts with phosphoric acid to form a nucleotide. "The structural moiety of the oligonucleotide comprises the nucleotide comprising the modified nucleoside compound according to the first aspect" means that the nucleoside moiety of at least one nucleotide in the oligonucleotide sequence is provided by the modified nucleoside compound provided by the present disclosure. That is, among the several nucleotides comprised in the oligonucleotide, the nucleoside moiety of at least one nucleotide is replaced by the modified nucleoside compound provided by the present disclosure.

In some preferred embodiments, for oligonucleotides with a length of no more than 30 nucleotides (*e.g.*, 15-30 nucleotides), the number of structural moieties (nucleotides) comprising the modified nucleoside compound provided by the present disclosure does not exceed 3 *(e.g.,* it may be 1, 2, or 3).

According to some preferred embodiments of the present disclosure, the nucleotide comprising the modified nucleoside compound is located at the 5' end of the oligonucleotide.

According to a particularly preferred embodiment of the present disclosure, the last nucleotide at the 5' end of the oligonucleotide is a nucleotide comprising the modified nucleoside compound.

According to some preferred embodiments of the present disclosure, the oligonucleotide is an oligonucleotide represented by formula (II) or a pharmaceutically acceptable salt thereof, 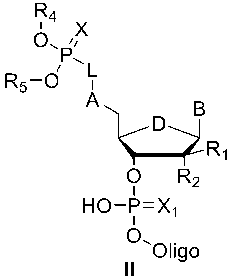 wherein
Oligo is an oligonucleotide,
R₁ and R₂ are each independently selected from H, halogen, C₁₋₆ alkyl, and C₁₋₆ alkoxy;
R₄ and R₅ are each independently selected from H, C₁₋₆ alkyl, -CH₂CH₂CN, and - CH₂O(CO)C(CH₃)₃;
X is O or S;
L is C₁₋₆ alkyl, C₂₋₅ alkenyl, or C₂₋₅ alkynyl;
A is O or S;
D is O, S, or -CH₂;
X₁ is O or S, and B is 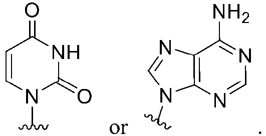 It should be noted that the structure shown in the above formula (II) is an oligonucleotide where the last nucleotide at the 5' end is a nucleotide comprising the modified nucleoside compound provided by the present disclosure; that is, the part connected to Oligo is the structure of the last nucleotide in the complete oligonucleotide, while Oligo represents the remaining moiety of the oligonucleotide starting from the second nucleotide at the 5' end.

After the modified nucleoside compound provided by the present disclosure replaces part of the nucleoside structures in an oligonucleotide, the oligonucleotide can become more stable, and the function of the oligonucleotide itself can be promoted and enhanced. Any oligonucleotide known in the art can be modified using the modified nucleoside compound provided by the present disclosure (that is, the modified nucleoside compound is used to replace the nucleoside structural moiety of part of the nucleotides in the oligonucleotide). According to a preferred embodiment of the present disclosure, the oligonucleotide is selected from any one of a small interfering RNA (siRNA), an antisense oligonucleotide (ASO), microRNA (miRNA), a small activating RNA (saRNA), a small guide RNA (sgRNA), a transfer RNA (tRNA), and an aptamer, or a combination of at least two thereof.

In the present disclosure, the oligonucleotide may be a single-stranded oligonucleotide or a double-stranded oligonucleotide. Unless otherwise specified, in the present disclosure, the length of an oligonucleotide refers to the number of nucleotides (also referred to as the number of bases, nt) for a single-stranded oligonucleotide, and the number of nucleotide pairs (also referred to as the number of base pairs, bp) for a double-stranded oligonucleotide.

According to some preferred embodiments of the present disclosure, the oligonucleotide is an siRNA.

Preferably, the siRNA is a double-stranded siRNA or a single-stranded siRNA.

According to some particularly preferred embodiments of the present disclosure, the siRNA is a double-stranded siRNA comprising a sense strand and an antisense strand.

Preferably, the antisense strand in the double-stranded siRNA is the oligonucleotide represented by formula (II) or a pharmaceutically acceptable salt thereof.

According to preferred embodiments of the present disclosure, each nucleotide in the oligonucleotide is independently a modified or unmodified nucleotide.

According to some preferred embodiments of the present disclosure, the oligonucleotide can be used to regulate the expression of a target gene *(e.g.,* to inhibit the expression of a target gene).

According to some particularly preferred embodiments of the present disclosure, the oligonucleotide is any one of the following siRNAs or a combination of at least two thereof:
(i) the sense chain has a sequence as shown in SEQ ID NO: 5, and the antisense strand has a sequence as shown in SEQ ID NO: 6;
(ii) the sense chain has a sequence as shown in SEQ ID NO: 7, and the antisense strand has a sequence as shown in SEQ ID NO: 8;
(iii) the sense chain has a sequence as shown in SEQ ID NO: 9, and the antisense strand has a sequence as shown in SEQ ID NO: 10;
(iv) the sense chain has a sequence as shown in SEQ ID NO: 15, and the antisense strand has a sequence as shown in SEQ ID NO: 16;
(v) the sense chain has a sequence as shown in SEQ ID NO: 17, and the antisense strand has a sequence as shown in SEQ ID NO: 18;
(vi) the sense chain has a sequence as shown in SEQ ID NO: 19, and the antisense strand has a sequence as shown in SEQ ID NO: 20.

The third aspect of the present disclosure provides a nucleic acid conjugate, wherein the nucleic acid conjugate comprises the oligonucleotide according to the second aspect or a pharmaceutically acceptable salt thereof, and a targeting ligand conjugated thereto.

In the present disclosure, any targeting ligand known in the art to be usable for nucleic acid conjugates may be selected. According to some preferred embodiments of the present disclosure, the targeting ligand comprises any one of a carbohydrate, cholesterol, a lipid, a polypeptide, or an antibody, or a combination of at least two thereof.

Preferably, the targeting ligand comprises an N-acetylgalactosamine (GalNAc) moiety.

The GalNAc moieties commonly used in the art as targeting ligands for nucleic acid conjugates are all applicable to the present disclosure. According to some preferred embodiments of the present disclosure, the GalNAc moiety is a monovalent GalNAc moiety, a divalent GalNAc moiety, a trivalent GalNAc moiety, or a tetravalent GalNAc moiety.

According to some particularly preferred embodiments of the present disclosure, the targeting ligand is G5, the structure of which is as follows: 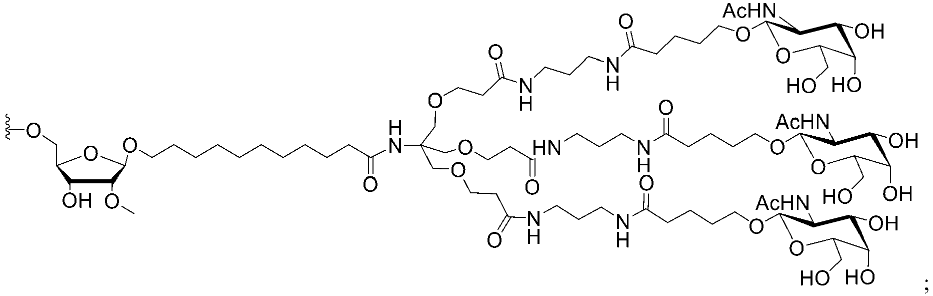 wherein 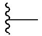 represents connection to the 3' end of the sense strand of siRNA through a phosphate group or a thiophosphate group.

The fourth aspect of the present disclosure provides a pharmaceutical composition, wherein the pharmaceutical composition comprises the oligonucleotide according to the second aspect or a pharmaceutically acceptable salt thereof;
and/or, the pharmaceutical composition comprises the nucleic acid conjugate according to the third aspect.

In the pharmaceutical composition provided by the present disclosure, the oligonucleotide and/or nucleic acid conjugate provided by the present disclosure serves as the main active ingredient. In some embodiments, the oligonucleotide and/or nucleic acid conjugate provided by the present disclosure can serve as the sole active ingredient in the pharmaceutical composition; in some embodiments, in addition to the oligonucleotide and/or nucleic acid conjugate of the present disclosure, the pharmaceutical composition may further comprise other active ingredients.

According to some preferred embodiments of the present disclosure, the pharmaceutical composition further comprises a pharmaceutically acceptable excipient. The "pharmaceutically acceptable excipient" refers to any non-active ingredient material that can be added during the process. For example, the pharmaceutical composition provided by the present disclosure may comprise pharmaceutically acceptable carriers, adjuvants, excipients, and the like, and may further comprise other active ingredients or active ingredient auxiliaries. The term "pharmaceutically acceptable carrier" refers to pharmaceutically acceptable materials, compositions, or carriers, such as liquid or solid fillers, stabilizers, dispersants, suspending agents, diluents, excipients, thickeners, solvents, or encapsulating materials, which are involved in carrying or delivering the nucleoside analogs, oligonucleotides, conjugates, or compositions thereof of the present disclosure in or to a patient, thereby enabling them to perform their intended functions. Typically, such constructs are carried or transported from one organ or part of the body to another organ or part of the body. Each carrier must be "acceptable", meaning that it is compatible with the other ingredients of the formulation, including the nucleoside analogs, oligonucleotides, conjugates of the present disclosure, or compositions thereof, and is harmless or substantially harmless to the patient. Some examples of materials that can be used as pharmaceutically acceptable carriers include: sugars, such as lactose, glucose, and sucrose; starches, such as corn starch and potato starch; cellulose and its derivatives, for example, sodium carboxymethyl cellulose, ethyl cellulose, and cellulose acetate; powdered tragacanth; malt; gelatin; talc; excipients such as cocoa butter and suppository wax; oils, such as peanut oil, cottonseed oil, safflower oil, sesame oil, olive oil, corn oil, and soybean oil; glycols, such as propylene glycol; polyols, such as glycerin, sorbitol, mannitol, and polyethylene glycol; esters, such as ethyl oleate and ethyl laurate; agar; buffers, such as magnesium hydroxide and aluminum hydroxide; surfactants; alginic acid; pyrogen-free water; isotonic saline; Ringer's solution; ethanol; phosphate-buffered solutions; and other non-toxic, compatible substances used in pharmaceutical formulations. As used herein, "pharmaceutically acceptable carrier" further includes any and all coatings, antibacterial and antifungal agents, and absorption delaying agents, and the like, that are compatible with the active nucleoside analogs, oligonucleotides, conjugates of the present disclosure, or compositions thereof, and are physiologically acceptable to the subject. Supplementary active compounds may also be incorporated into the composition. The "pharmaceutically acceptable carrier" may further include pharmaceutically acceptable salts of compounds that can be used in the present disclosure. Other additional components that may be included in the pharmaceutical compositions used in the practice of the present disclosure are known in the art and are described, for example, in Remington's Pharmaceutical Sciences (Genaro, Ed., Mack Publishing Co., 1985, Easton, PA), which is incorporated herein by reference. Any carrier, adjuvant, or excipient known in the art for use in pharmaceuticals can be suitable for the present disclosure. For example, the excipient may include, but is not limited to, binders, suspending agents, emulsifiers, diluents (or fillers), granulating agents, adhesives, disintegrants, lubricants, anti-adherents, wetting agents, gelling agents, absorption delaying agents, dissolution inhibitors, enhancers, adsorbents, chelating agents, colorants, flavoring agents, coating agents, buffers, protective agents, preservatives, solubilizing agents, pH regulators, and the like.

The fifth aspect of the present disclosure provides the use of the oligonucleotide according to the second aspect or a pharmaceutically acceptable salt thereof, or the nucleic acid conjugate according to the third aspect, or the pharmaceutical composition according to the fourth aspect in the manufacture of a medicament for treating and/or preventing a pathological condition or disease caused by expression of the proprotein convertase subtilisin/kexin type 9 gene *(PCSK9* gene) in hepatocytes.

The *PCSK9* gene is a gene located on chromosome 1, and the protein encoded thereby plays an important role in cholesterol and fatty acid metabolism, and aberrant expression of this gene is associated with autosomal dominant familial hypercholesterolemia and other metabolic diseases.

According to some preferred embodiments of the present disclosure, the pathological condition or disease is selected from any one of hypercholesterolemia, dyslipidemia, atherosclerosis, and cardiovascular or cerebrovascular diseases, or a combination of at least two thereof.

Furthermore, the present disclosure also provides a method of treating a pathological condition or disease caused by expression of the *PCSK9* gene, the method comprising administering to a subject in need thereof the oligonucleotide according to the second aspect of the present disclosure or a pharmaceutically acceptable salt thereof, or the nucleic acid conjugate according to the third aspect, or the pharmaceutical composition according to the fourth aspect, or administering a medicament prepared from the oligonucleotide according to the second aspect of the present disclosure or a pharmaceutically acceptable salt thereof, or the nucleic acid conjugate according to the third aspect, or the pharmaceutical composition according to the fourth aspect.

According to some preferred embodiments of the present disclosure, the subject may be a mammal. For example, the subject may be a human, a non-human primate, a rodent (such as a rabbit, rat, mouse, and guinea pig), a horse, a sheep, a pig, a cat, a dog, and the like.

The sixth aspect of the present disclosure provides a kit, comprising the oligonucleotide according to the second aspect or a pharmaceutically acceptable salt thereof, or the nucleic acid conjugate according to the third aspect, or the pharmaceutical composition according to the fourth aspect.

The kit provided by the present disclosure can be used for therapeutic or non-therapeutic uses, and can also be used for diagnostic or non-diagnostic uses.

The positive and progressive effect of the present disclosure lies in that a series of 5'-phosphate modified nucleosides are designed, whose chemical structures are completely different from those of compounds phosphorylated at the 5' end in the prior art. Oligonucleotides comprising the 5'-phosphate modified nucleosides of the present disclosure can significantly enhance the inhibitory activity of siRNA against target genes.

Compared with the prior art, the present disclosure has at least the following beneficial effects:

### 1. The compound structure used for 5'-end phosphate modification is completely different from that in existing technologies.

In the 5'-phosphate modified nucleoside designed in the present disclosure, an alkyl group is introduced between the phosphorus atom of the phosphate and the 5'-hydroxyl group of the sugar ring, which is completely different from the prior art and constitutes an entirely new 5'-phosphate modified nucleoside compound. This compound is prepared from commercially available nucleosides as starting materials, where the 5'-hydroxyl group of the nucleoside is reacted with alkyl/alkenyl compounds containing phosphates, so that a series of 5'-phosphate modified nucleoside compounds can be rapidly constructed through only a single nucleophilic substitution/addition reaction.

### 2. Compared with compounds in the prior art, the 5'-phosphate modified siRNA sequences of the present disclosure have higher inhibitory activity.

For example, compared with D84-DV27-PG5 (compound in the prior art modified with (E)-VP-Um), the inhibitory rates of D84-DV27-5MMPG5 (compound modified with YK-VP-002 of the present disclosure) on PCSK9 protein expression in mouse serum on Day 7, Day 14, and Day 21 are increased by 11.7%, 7.3%, and 9.8%, respectively. Compared with D84-DV27-4MMPG5 (compound in the prior art modified with Phosphoramidite 3), the inhibitory rates of D84-DV27-5MMPG5 (compound modified with YK-VP-002 of the present disclosure) on PCSK9 protein expression in mouse serum on Day 7, Day 14, and Day 21 are increased by 11.8%, 16.5%, and 15.3%, respectively.

Compared with D84-DV27-PG5, the reduction levels of LDL-C in mouse serum on Day 7, Day 14, and Day 21 by D84-DV27-5EMPG5 (compound modified with YK-VP-001 of the present disclosure) are increased by 9.9%, 8.5%, and 8.4%, respectively. Compared with D84-DV27-4MMPG5, the reduction levels of D84-DV27-5MMPG5 on Day 7, Day 14, and Day 21 are increased by 11.2%, 11.6%, and 16.0%, respectively.

### BRIEF DESCRIPTION OF THE DRAWINGS

In order to more clearly illustrate the technical solutions in the detailed description of the preferred embodiment of the present disclosure or in the prior art, the drawings required to be used in the description of the detailed description of the preferred embodiment or the prior art are briefly introduced below. It is obvious that the drawings in the following description are some embodiments of the present disclosure, and for those of ordinary skill in the art, other drawings can also be obtained according to these drawings without creative efforts.

FIG. 1 shows the inhibitory rates of PCSK9 protein expression in mouse serum on Day 7, Day 14, and Day 21 after administration of siRNA sequences D84-DV27-G5, D84-DV27-PG5, D84-DV27-5EMPG5, D84-DV27-5MMPG5, D84-DV27-5MEPG5, D84-DV27-4MMPG5, D82-DV29-G5, D82-DV29-5MMPG5, D82-DV29-5EMPG5, and D82-DV29-5MPG5.

FIG. 2 shows the reduction levels of LDL-C in mouse serum on Day 7, Day 14, and Day 21 after administration of siRNA sequences D84-DV27-G5, D84-DV27-PG5, D84-DV27-5EMPG5, D84-DV27-5MMPG5, D84-DV27-5MEPG5, D84-DV27-4MMPG5, D82-DV29-G5, D82-DV29-5MMPG5, D82-DV29-5EMPG5, and D82-DV29-5MPG5.

### DETAILED DESCRIPTION OF THE PREFERRED EMBODIMENT

In order to make the objectives, technical solutions, and advantages of the examples of the present disclosure clearer, the technical solutions of the examples of the present disclosure will be clearly and fully described below. Apparently, the examples described herein constitute only part of the examples of the present disclosure, rather than all of the examples. Based on the examples of the present disclosure described herein, all other examples that can be obtained by those of ordinary skill in the art without inventive effort fall within the scope of protection of the present disclosure.

The present disclosure may be implemented in other specific forms without departing from its essential characteristics. It should be understood that, provided there is no conflict, any one and all embodiments of the present disclosure may be combined with the technical features of any other embodiment or multiple other embodiments to obtain additional embodiments. The present disclosure encompasses such additional embodiments obtained through these combinations.

All publications and patents referenced in the present disclosure are incorporated herein by reference in their entirety. If usage or terminology used in any publications and patents incorporated by reference conflicts with usage or terminology used in the present disclosure, the usage and terminology in the present disclosure shall prevail.

The section headings used herein are for the sole purpose of organizing the article and should not be construed as a limitation on the subject matter described.

Unless otherwise specified, all technical and scientific terms used herein have their ordinary meanings in the art to which the claimed subject matter pertains. If there are multiple definitions for a term, the definition herein shall prevail.

Except in the working examples or otherwise indicated, all numbers stating quantitative properties, such as doses, in the specification and claims should be understood as modified in all instances by the term "about". It should also be understood that any numerical ranges recited in the present disclosure are intended to include all sub-ranges within such ranges and any combination of the respective endpoints of such ranges or sub-ranges.

The words "comprising", "containing", or "including" and the like, as used herein, mean that the element appearing before the word covers the elements listed after the word and their equivalents, and does not exclude unrecited elements. The term "containing" or "comprising (including)" as used herein may be open, semi-closed, or closed. In other words, these terms also encompass "consisting essentially of ..." or "consisting of ...".

In the present disclosure, "protecting group" refers to a group used, in the conventional chemical sense, to reversibly render a functional group unreactive under certain conditions of a desired reaction. After the desired reaction, the protecting group can be removed to deprotect the protected functional group. All protecting groups should be removable under conditions that do not degrade a significant proportion of the molecules being synthesized.

In the present disclosure, the "phosphorus-containing active reactive group" refers to a phosphorus-containing group that can undergo a nucleophilic reaction with a hydroxyl or amino group contained in another molecule, in particular in another nucleotide moiety or another nucleotide analog. Typically, such a reaction produces an ester-type internucleosidic linkage connecting a nucleotide moiety or nucleotide analogue moiety with another nucleotide moiety or nucleotide analogue moiety. These phosphorus-containing active reactive groups are known in the art and contain phosphorus atoms in a P(III) or P(V) valence state. The phosphorus-containing active reactive groups include, but are not limited to, phosphoramidites, H-phosphonates, phosphotriesters, and phosphorus-containing chiral auxiliaries, such as 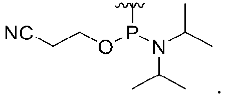 Among them, the phosphorus-containing chiral auxiliary refers to a phosphorus-containing group having chirality, which may be a group in any of various possible forms such as

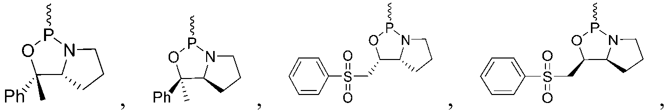

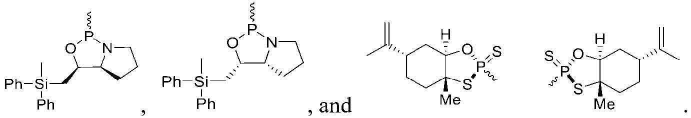

The term "halogen" refers to fluorine, chlorine, bromine, or iodine.

The term "C₁₋₆" refers to carbon atoms having any integer value in the range of 1 to 6 in total in the main chain and side chains of a group, for example, 1, 2, 3, 4, 5, or 6 carbon atoms. Similarly, the term "C₂₋₅" refers to carbon atoms having any integer value in the range of 2 to 5 in total in the main chain and side chains of a group, for example, 2, 3, 4, or 5 carbon atoms.

The term "alkyl" refers to a straight-chain or branched alkyl group having the specified number of carbon atoms (for example, C₁₋₆). The alkyl includes, but is not limited to, methyl, ethyl, *n*-propyl, isopropyl, *n*-butyl, *tert*-butyl, isobutyl, *sec*-butyl, *n*-pentyl, *n*-hexyl, and the like. The term "alkyl" also includes heteroalkyl, namely a group formed by replacing one or more (*e.g*., 1, 2, 3, or 4) carbon atoms in an alkyl group with heteroatoms (that is, atoms other than carbon or hydrogen, such as oxygen, sulfur, fluorine, nitrogen, and phosphorus).

The term "alkoxy" refers to a group R-O-, wherein R is an alkyl group as defined above.

The term "alkenyl" refers to a hydrocarbon group that comprises at least one carbon-carbon double bond at one or more positions along the carbon chain of an alkyl group, including but not limited to vinyl, propenyl, butenyl, and the like.

The term "alkynyl" refers to a hydrocarbon group that comprises at least one carbon-carbon triple bond at one or more positions along the carbon chain of an alkyl group, including but not limited to ethynyl, propynyl, butynyl, and the like.

The term "pharmaceutically acceptable salt" refers to a salt obtained by the reaction of a compound with a pharmaceutically acceptable (relatively non-toxic, safe, and suitable for use in a subject) acid or base. When a compound contains a relatively acidic functional group, a base addition salt can be obtained by contacting the free form of the compound with an appropriate amount of a pharmaceutically acceptable base in a suitable inert solvent. Pharmaceutically acceptable base addition salts include, but are not limited to, sodium salts, potassium salts, calcium salts, aluminum salts, magnesium salts, bismuth salts, ammonium salts, and the like. When a compound contains a relatively basic functional group, an acid addition salt can be obtained by contacting the free form of the compound with an appropriate amount of a pharmaceutically acceptable acid in a suitable inert solvent. Pharmaceutically acceptable acid addition salts include, but are not limited to, hydrochlorides, sulfates, methanesulfonates, and the like.

The term "pharmaceutically acceptable excipient" in the present disclosure refers to all substances contained in a pharmaceutical formulation other than the active ingredient.

The term "treating" refers to any of the following: (1) alleviating one or more biological manifestations of a disease; (2) interfering with one or more points in the biological cascade that causes the disease; or (3) slowing the progression of one or more biological manifestations of the disease.

The term "preventing" refers to reducing the risk of occurrence of a disease or reducing the severity of the disease once it occurs.

The oligonucleotides in the present disclosure include single-stranded oligonucleotides (for example, antisense oligonucleotides, abbreviated as ASOs) and double-stranded oligonucleotides (for example, small interfering RNAs, abbreviated as siRNAs).

The oligonucleotides in the present disclosure include natural oligonucleotides and chemically modified oligonucleotides. The chemical modifications described herein include nucleoside modifications (including sugar moiety modifications and nucleobase modifications) and internucleoside linkage modifications. The chemical modifications of the oligonucleotides do not include situations in which the only differences lie in the nucleobase sequence. The "natural" as used herein refers to the case corresponding to naturally occurring RNA or DNA.

On the basis of common sense in the art, the above preferred conditions can be combined arbitrarily to obtain preferred examples of the present disclosure.

All reagents and starting materials used herein are commercially available.

### Example

In order to make the objectives, technical solutions, and advantages of the examples of the present disclosure clearer, the technical solutions of the examples of the present disclosure will be clearly and fully described below. Apparently, the examples described herein constitute only part of the examples of the present disclosure, rather than all of the examples. Based on the examples of the present disclosure described herein, all other examples that can be obtained by those of ordinary skill in the art without inventive effort fall within the scope of protection of the present disclosure.

The present disclosure may be implemented in other specific forms without departing from its essential characteristics. It should be understood that, provided there is no conflict, any one and all embodiments of the present disclosure may be combined with the technical features of any other embodiment or multiple other embodiments to obtain additional embodiments. The present disclosure encompasses such additional embodiments obtained through these combinations.

The present disclosure is further described below in conjunction with the examples. However, the present disclosure is not limited to the following examples. The implementation conditions used in the examples may be further adjusted according to different requirements of specific use, and the implementation conditions not specified are conventional conditions in the art. In the specific examples of the present disclosure, all starting materials used are commercially available. Unless otherwise specified, all temperatures are given in degrees Celsius. The technical features involved in various embodiments of the present disclosure may be combined with each other as long as they do not conflict with each other.

### Example 1: Synthesis of compounds

The following abbreviations respectively represent the following reagents: TBDMSCl: *tert*butyldimethylsilyl chloride; DCM: dichloromethane; MeOH: methanol; NaH: sodium hydride; PE: petroleum ether; EA: ethyl acetate; TEA: triethylamine; Et₃SiH: triethylsilane; TCA: trichloroacetic acid; DCI: 4,5-dicyanoimidazole.

### 1. Synthesis of YK-VP-001

The synthetic route is as follows:

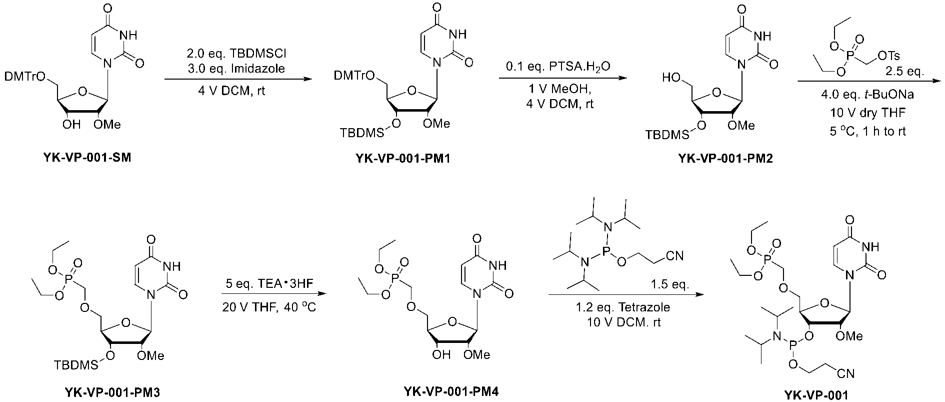

### Step 1: Synthesis of YK-VP-001-PM1

YK-VP-001-SM (365.00 g, 0.64 mol) was dissolved in 1460 mL of ultra-dry dichloromethane and stirred in an ice-water bath under a nitrogen atmosphere. Imidazole (130.70 g, 1.92 mol) and TBDMSCl (192.90 g, 1.28 mol) were then added in portions, and the mixture was stirred at room temperature for 5 hours under a nitrogen atmosphere. The reaction was monitored by TLC, and when the starting material disappeared and a new, less polar spot appeared, the reaction was stopped. Water (730 mL) was added to quench the reaction, followed by extraction, and then the organic phase was washed once with 730 mL of water. The organic phase was dried over anhydrous sodium sulfate, and the filtrate obtained after filtration was used directly for the next step.

### Step 2: Synthesis of YK-VP-001-PM2

Methanol (1 V, 365 mL) and *p*-toluenesulfonic acid monohydrate (12.20 g) were successively added to a dichloromethane (4 V, 1460 mL) solution of YK-VP-001-PM1 (0.64 mol), and the mixture was stirred at room temperature. The reaction was monitored by TLC, and the starting material disappeared after 2 hours. TEA (182 mL, 0.5 V) was added to quench the reaction, and the mixture was stirred for 1 hour, followed by the addition of saturated aqueous sodium bicarbonate solution (730 mL) and further stirring for 15 minutes. The phases were then separated, and the aqueous phase was extracted once with dichloromethane (365 mL). The combined organic phases were washed once with saturated aqueous sodium chloride solution (730 mL), dried over anhydrous sodium sulfate, filtered, and concentrated by rotary evaporation to obtain a light yellow solid. The crude product was triturated in n-heptane (1460 mL, 4 V), and the mixture was filtered to obtain 183.20 g of a white solid with a yield of 75.5%. MS m/z [M + H]⁺ = 373.0. ¹H NMR (400 MHz, CDCl₃) δ 9.68 (s, 1H), 7.74 (d,*J* = 8.1 Hz, 1H), 5.75 - 5.67 (m, 2H), 4.33 (t, *J =* 5.3 Hz, 1H), 4.07 - 4.01 (m, 1H), 4.00 - 3.89 (m, 2H), 3.80 - 3.68 (m, 1H), 3.47 (s, 3H), 3.08 (dd, *J* = 6.6, 3.6 Hz, 1H), 0.89 (s, 9H), 0.08 (d, *J* = 5.0 Hz, 6H).

### Step 3: Synthesis of YK-VP-001-PM3

YK-VP-001-PM2 (20.00 g, 53.70 mmol) was dissolved in 200 mL of ultra-dry tetrahydrofuran, and sodium tert-butoxide (20.64 g, 214.80 mmol) was added at 5°C under a nitrogen atmosphere, followed by stirring for 1 hour and then stirring at room temperature for an additional 1 hour. Diethyl (tosyloxy)methylphosphonate (43.26 g, 134.20 mmol) was added, and the reaction was stirred at room temperature for 2 hours. The reaction was monitored by TLC, and when the starting material was nearly consumed with a new spot appearing below it, the reaction was stopped. The reaction mixture was diluted with 500 mL of ethyl acetate, then quenched by adding 500 mL of saturated aqueous ammonium chloride solution. The phases were then separated, and the aqueous phase was extracted once with ethyl acetate (500 mL). The combined organic phases were washed once with saturated aqueous sodium chloride solution (500 mL), dried over anhydrous sodium sulfate, filtered, and concentrated by rotary evaporation to obtain a crude product. The crude product was purified by medium-pressure column chromatography (SiO₂, PE/EA = 1/3) to obtain 13.10 g of a light yellow oil with a yield of 46.4%. MS m/z [M - H]⁻ = 521.2. ¹H NMR (400 MHz, CDCl₃) δ 9.77 (s, 1H), 8.01 (d, *J* = 8.1 Hz, 1H), 5.91 (d, *J* = 2.0 Hz, 1H), 5.78 (dd, *J=* 8.1, 1.8 Hz, 1H), 4.24 - 4.10 (m, 5H), 4.09 - 4.04 (m, 1H), 3.94 (dd, *J=* 10.8, 2.2 Hz, 1H), 3.89 - 3.74 (m, 2H), 3.67 (dd, *J=* 10.9, 1.9 Hz, 1H), 3.60 (dd, *J =* 4.8, 2.1 Hz, 1H), 3.52 (s, 3H), 1.32 (td, *J=* 7.1, 4.5 Hz, 6H), 0.87 (s, 9H), 0.07 (d, *J =* 5.3 Hz, 6H). ³¹P NMR (162 MHz, CDCl₃) δ 21.25.

### Step 4: Synthesis of YK-VP-001-PM4

YK-VP-001-PM3 (12.00 g, 23.00 mmol) was dissolved in 240 mL of tetrahydrofuran, and triethylamine trihydrofluoride (18.51 g, 114.80 mmol) was added. The mixture was stirred at 40°C for 19 hours. The reaction was monitored by TLC and LC/MS, and when the starting material disappeared and the target molecular weight was detected, the reaction was stopped. The reaction mixture was directly concentrated by rotary evaporation, and then acetonitrile was added, followed by another concentration to obtain a crude product. The crude product was purified by medium-pressure column chromatography (SiO₂, DCM/MeOH = 20/1) to obtain 9.30 g of a light yellow oil with a yield of 99.14%. MS m/z [M + H]⁺ = 409.0. ¹H NMR (400 MHz, CDCl₃) δ 9.13 (s, 1H), 7.97 (d, *J* = 8.1 Hz, 1H), 5.98 (d, *J* = 2.5 Hz, 1H), 5.78 (dd, *J* = 8.2, 1.2 Hz, 1H), 4.28 (dd, *J* = 6.8, 5.1 Hz, 1H), 4.19 - 4.11 (m, 4H), 4.05 (dt, *J=* 6.9, 2.1 Hz, 1H), 3.99 (dd, *J=* 10.8, 2.2 Hz, 1H), 3.90 - 3.74 (m, 4H), 3.58 (s, 3H), 3.46 (s, 1H), 1.35 - 1.34 (m, 6H). ³¹P NMR (162 MHz, CDCl₃) δ 21.54.

### Step 5: Synthesis of YK-VP-001

YK-VP-001-PM4 (3.00 g, 7.30 mmol) was co-evaporated on a rotary evaporator with ultra-dry dichloromethane three times. The dried YK-VP-001-PM4 was dissolved in ultra-dry dichloromethane (30 mL), cooled to 5°C under a nitrogen atmosphere, and then 2-cyanoethyl N,N,N',N'-tetraisopropylphosphorodiamidite (3.32 g, 11.00 mmol) and tetrazole (0.62 g, 8.80 mmol) were added successively. The mixture was stirred at room temperature for 1 hour under a nitrogen atmosphere. The reaction was monitored by LC/MS and TLC, and when the target molecular weight was detected, the reaction was stopped. The reaction mixture was washed twice with saturated aqueous sodium bicarbonate solution (15 mL × 2). The aqueous phase was then extracted once with 30 mL of dichloromethane. The combined organic phases were washed once with 10% aqueous sodium chloride solution, dried over anhydrous sodium sulfate, filtered, and concentrated by rotary evaporation to obtain a crude product. After rapid purification, 1.80 g of colorless oil was obtained with a yield of 40.5%. MS m/z [M - H]⁻ = 607.5. ¹H NMR (400 MHz, CDCl₃) δ 9.22 (s, 1H), 7.94 (dd, *J=* 9.1, 5.7 Hz, 1H), 6.00 - 5.97 (m, 1H), 5.78 (dd, *J =* 8.2, 3.7 Hz, 1H), 4.49 - 4.04 (m, 6H), 3.98 - 3.73 (m, 6H), 3.72 - 3.55 (m, 3H), 3.49 - 3.42 (m, 3H), 2.65 (m, 2H), 1.32 - 1.26 (m, 6H), 1.19 - 1.10 (m, 12H). ³¹P NMR (162 MHz, CDCl₃) δ 151.41, 150.47, 21.49, 21.25.

### 2. Synthesis of YK-VP-002

The synthetic route is as follows:

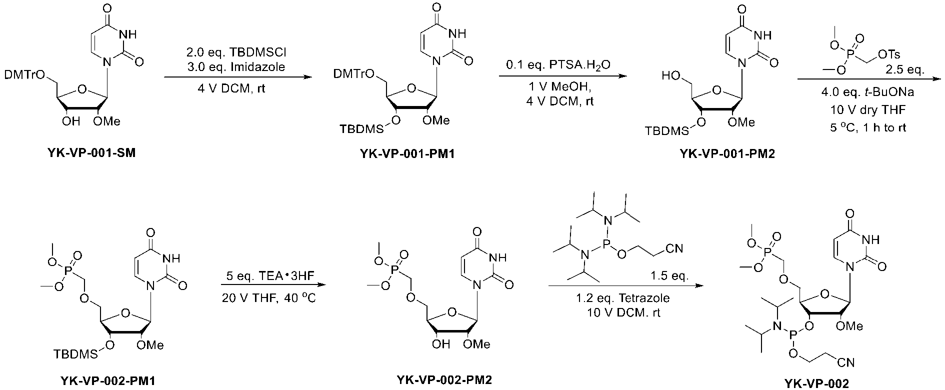

### Step 1: Synthesis of YK-VP-002-PM1

YK-VP-001-PM2 (34.00 g, 91.20 mmol) was dissolved in 340 mL of ultra-dry tetrahydrofuran, and sodium *tert*-butoxide (35.08 g, 364.80 mmol) was added at 5°C under a nitrogen atmosphere, followed by stirring for 1 hour and then stirring at room temperature for an additional 1 hour. Dimethyl (tosyloxy)methylphosphonate (67.14 g, 228.00 mmol) was added, and the reaction was stirred at room temperature for 2 hours. The reaction was monitored by TLC, and when the starting material was nearly consumed with a new spot appearing below it, the reaction was stopped. The reaction mixture was diluted with 500 mL of ethyl acetate, then quenched by adding 500 mL of saturated aqueous ammonium chloride solution. The phases were then separated, and the aqueous phase was extracted once with ethyl acetate (500 mL). The combined organic phases were washed once with saturated aqueous sodium chloride solution (500 mL), dried over anhydrous sodium sulfate, filtered, and concentrated by rotary evaporation to obtain a crude product. The crude product was purified by medium-pressure column chromatography (SiO₂, PE/EA = 1/8) to obtain 12.00 g of a light yellow oil with a yield of 26.58%. MS m/z [M + NH₄]⁺ = 512.2. ¹HNMR (400 MHz, CDCl₃) δ 8.69 (s, 1H), 7.99 (dd, *J =* 8.1, 3.8 Hz, 1H), 5.91 (d, *J=* 2.3 Hz, 1H), 5.77 (d, *J* = 8.1 Hz, 1H), 4.26 - 4.22 (m, 1H), 4.09 - 4.07 (m, 1H), 3.94 (dd, *J =* 11.1, 2.4 Hz, 1H), 3.89 - 3.73 (m, 8H), 3.68 (dd, *J =* 10.7, 1.9 Hz, 1H), 3.61 - 3.60 (m, 1H), 3.54 - 3.53 (m, 3H), 0.90 - 0.88 (m, 9H), 0.10 - 0.06 (m, 6H). ³¹P NMR (162 MHz, CDCl₃) δ 23.69.

### Step 2: Synthesis of YK-VP-002-PM2

YK-VP-002-PM1 (12.00 g, 24.20 mmol) was dissolved in 240 mL of tetrahydrofuran, and triethylamine trihydrofluoride (19.56 g, 126.00 mmol) was added. The mixture was stirred at 40°C for 19 hours. The reaction was monitored by TLC and LC/MS, and when the starting material disappeared and the target molecular weight was detected, the reaction was stopped. The reaction mixture was directly concentrated by rotary evaporation, and then acetonitrile was added, followed by another concentration to obtain a crude product. The crude product was purified by medium-pressure column chromatography (SiO₂, DCM/MeOH = 50/1) to obtain 4.80 g of a light yellow solid with a yield of 52.0%. MS m/z [M + NH₄]⁺ = 398.0. ¹H NMR (400 MHz, DMSO-*d*₆) δ 11.37 (s, 1H), 7.82 (d, *J* = 8.1 Hz, 1H), 5.86 (d, *J* = 4.8 Hz, 1H), 5.62 (d, *J =* 8.1 Hz, 1H), 5.31 (d, *J* = 5.0 Hz, 1H), 4.10 (d, *J* = 4.6 Hz, 1H), 3.97 - 3.95 (m, 3H), 3.82 - 3.73 (m, 2H), 3.72 - 3.64 (m, 7H), 3.35 (s, 3H). ³¹P NMR (162 MHz, DMSO-*d*₆) δ 24.49.

### Step 3: Synthesis of YK-VP-002

YK-VP-002-PM2 (1.60 g, 4.20 mmol) was co-evaporated on a rotary evaporator with ultra-dry dichloromethane three times. The dried YK-VP-002-PM2 was dissolved in ultra-dry dichloromethane (16 mL), cooled to 5°C under a nitrogen atmosphere, and then 2-cyanoethyl N,N,N',N'-tetraisopropylphosphorodiamidite (1.90 g, 6.30 mmol) and tetrazole (0.35 g, 5.04 mmol) were added successively. The mixture was stirred at room temperature for 1 hour under a nitrogen atmosphere. The reaction was monitored by LC/MS and TLC, and when the target molecular weight was detected, the reaction was stopped. The reaction mixture was washed twice with saturated aqueous sodium bicarbonate solution (15 mL × 2). The aqueous phase was then extracted once with 30 mL of dichloromethane. The combined organic phases were washed once with 10% aqueous sodium chloride solution, dried over anhydrous sodium sulfate, filtered, and concentrated by rotary evaporation to obtain a crude product. After rapid purification, 1.60 g of colorless oil was obtained with a yield of 65.6%. MS m/z [M - H]⁻ = 579.4. ¹H NMR (400 MHz, DMSO-*d*₆) δ 11.39 (s, 1H), 7.84 (dd, *J* = 8.1, 3.2 Hz, 1H), 5.88 (t, *J=* 5.2 Hz, 1H), 5.64 (d, *J =* 8.1 Hz, 1H), 4.44 - 4.33 (m, 1H), 4.19 - 4.11 (m, 1H), 4.04 - 3.88 (m, 3H), 3.85 - 3.73 (m, 3H), 3.72 - 3.67 (m, 6H), 3.65 - 3.57 (m, 3H), 3.39 (s, 2H), 3.33 (s, 1H), 2.84 - 2.76 (m, 2H), 1.19 - 1.14 (m, 12H). ³¹P NMR (162 MHz, DMSO-*d*₆) δ 150.66, 149.83, 24.45, 24.34.

### 3. Synthesis of YK-VP-003

The synthetic route is as follows:

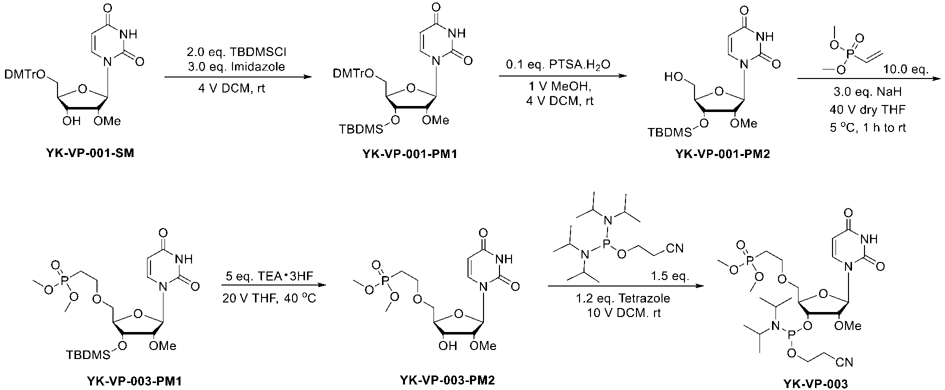

### Step 1: Synthesis of YK-VP-003-PM1

YK-VP-001-PM2 (6.50 g, 17.45 mmol) was dissolved in 105 mL of ultra-dry tetrahydrofuran, and NaH (2.09 g, 52.35 mmol) was added in portions at 5°C under a nitrogen atmosphere, followed by stirring for 1 hour and then stirring at room temperature for an additional 1 hour. Dimethyl vinylphosphonate (23.75 g, 174.50 mmol) was added in an ice-water bath, and the reaction was then stirred overnight at room temperature. The reaction was monitored by TLC and LC/MS, and when the starting material was essentially consumed and the target molecular weight was detected, the reaction was stopped. 300 mL of ethyl acetate was added for dilution, and the mixture was washed twice with saturated aqueous ammonium chloride solution (300 mL × 2). The combined aqueous phases were back-extracted once with 300 mL of ethyl acetate. The organic phase was dried over anhydrous sodium sulfate, filtered, and concentrated by rotary evaporation to obtain a crude product. The crude product was purified by medium-pressure column chromatography (SiO₂, PE/EA = 1/8) to obtain 4.02 g of a light yellow oil with a yield of 45.32%. MS m/z [M + NH₄]⁺ = 526.2. ¹H NMR (400 MHz, CDCl₃) δ 9.45 (s, 1H), 7.92 (d, *J =* 8.1 Hz, 1H), 5.87 (d, *J =* 1.9 Hz, 1H), 5.71 (d, *J =* 8.1 Hz, 1H), 4.20 (dd, *J =* 7.6, 4.8 Hz, 1H), 4.15 - 4.01 (m, 1H), 3.83 (dd, *J =* 10.9, 2.2 Hz, 1H), 3.80 - 3.70 (m, 8H), 3.61 (dd, *J=* 4.9, 2.1 Hz, 1H), 3.57 - 3.50 (m, 4H), 2.04 - 2.14 (m, 2H), 0.87 (s, 9H), 0.06 (d, *J=* 5.3 Hz, 6H). ³¹P NMR (162 MHz, CDCl₃) δ 31.05.

### Step 2: Synthesis of YK-VP-003-PM2

YK-VP-003-PM1 (4.00 g, 7.86 mmol) was dissolved in 80 mL of tetrahydrofuran, and triethylamine trihydrofluoride (6.34 g, 39.30 mmol) was added. The mixture was stirred at 40°C for 19 hours. The reaction was monitored by TLC and LC/MS, and when the starting material disappeared and the target molecular weight was detected, the reaction was stopped. The reaction mixture was directly concentrated by rotary evaporation, and then acetonitrile was added, followed by another concentration to obtain a crude product. The crude product was purified by medium-pressure column chromatography (SiO₂, DCM/MeOH = 30/1) to obtain 1.80 g of a light yellow solid with a yield of 58.2%. MS m/z [M + H]⁺ = 395.0. ¹H NMR (400 MHz, CDCl₃) δ 8.79 (s, 1H), 7.93 (d, *J =* 8.1 Hz, 1H), 5.95 (s, 1H), 5.73 (d, *J =* 8.1 Hz, 1H), 4.31 - 4.26 (m, 1H), 4.04 (d, *J=* 7.2 Hz, 1H), 3.90 - 3.73 (m, 11H), 3.69 (d, *J* = 9.7 Hz, 1H), 3.61 (s, 3H), 2.15 - 2.07 (m, 2H). ³¹P NMR (162 MHz, CDCl₃) δ 31.36.

### Step 3: Synthesis of YK-VP-003

YK-VP-003-PM2 (1.80 g, 4.56 mmol) was co-evaporated on a rotary evaporator with ultra-dry dichloromethane three times. The dried YK-VP-003-PM2 was dissolved in ultra-dry dichloromethane (18 mL), cooled to 5°C under a nitrogen atmosphere, and then 2-cyanoethyl N,N,N',N'-tetraisopropylphosphorodiamidite (2.06 g, 6.84 mmol) and tetrazole (0.38 g, 5.47 mmol) were added successively. The mixture was stirred at room temperature for 1 hour under a nitrogen atmosphere. The reaction was monitored by LC/MS and TLC, and when the target molecular weight was detected, the reaction was stopped. The reaction mixture was washed twice with saturated aqueous sodium bicarbonate solution (15 mL × 2). The aqueous phase was then extracted once with 30 mL of dichloromethane. The combined organic phases were washed once with 10% aqueous sodium chloride solution, dried over anhydrous sodium sulfate, filtered, and concentrated by rotary evaporation to obtain a crude product. After rapid purification, 1.20 g of a colorless oil was obtained with a yield of 44.3%. MS m/z [M - H]⁻ = 593.5. ¹H NMR (400 MHz, DMSO-*d*₆) δ 11.38 (s, 1H), 7.81 (dd, *J* = 8.2, 1.6 Hz, 1H), 5.87 (dd, *J* = 5.7, 4.2 Hz, 1H), 5.71 (dd, *J* = 8.1, 2.2 Hz, 1H), 4.42 - 4.33 (m, 1H), 4.17 - 4.02 (m, 2H), 3.82 - 3.76 (m, 2H), 3.71 - 3.51 (m, 12H), 3.39 (s, 2H), 3.33 (s, 1H), 2.80 (td, *J* = 5.8, 1.1 Hz, 2H), 2.20 - 2.10 (m, 2H), 1.17 - 1.13 (m, 12H). ³¹P NMR (162 MHz, DMSO-*d*₆) *δ* 150.42, 149.68, 31.90, 31.69.

### 4. Synthesis of YK-VP-004

The synthetic route is as follows:

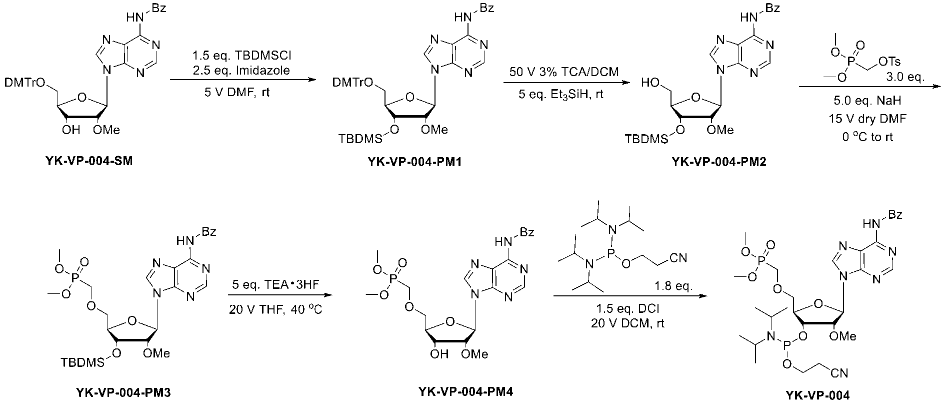

### Step 1: Synthesis of YK-VP-004-PM1

YK-VP-004-SM (20.00 g, 29.08 mmol) was dissolved in ultra-dry *N,N*-dimethylformamide (100 mL), followed by the portionwise addition of TBDMSCl (6.57 g, 43.62 mmol) and imidazole (4.95 g, 72.70 mmol). The mixture was stirred at room temperature under a nitrogen atmosphere for 21 hours. The reaction was monitored by TLC, and when the starting material disappeared and a new, less polar spot appeared, the reaction was stopped. Water was added to quench the reaction, followed by extraction with ethyl acetate (100 mL). The organic phase was washed with water four times (100 mL × 4), and the aqueous phase was back-extracted with ethyl acetate once (100 mL). The organic phase was dried over anhydrous sodium sulfate, filtered, and concentrated by rotary evaporation to obtain a crude product as a white solid (25.29 g), which was used directly in the next step. MS m/z [M + H]⁺ = 802.4.

### Step 2: Synthesis of YK-VP-004-PM2

YK-VP-004-PM1 (1.60 g, 2.00 mmol) was dissolved in 3% TCA/DCM (80 mL, 50 V), followed by the addition of Et₃SiH (1.60 mL, 10.00 mmol), and the mixture was stirred at room temperature for 2 hours. The reaction was monitored by TLC and LC/MS, and when the starting material disappeared and the target molecular weight was detected, the reaction was stopped. Water (80 mL) was added to quench the reaction, followed by extraction and two washes with dichloromethane (80 mL × 2). The organic phase was dried over anhydrous sodium sulfate, filtered, and concentrated by rotary evaporation to obtain an oil, which was purified by medium-pressure column chromatography (SiO₂, DCM/MeOH = 100/0 to 94/6) to obtain 0.89 g of a white solid with a yield of 89.6%. MS m/z [M + H]⁺ = 500.1. ¹H NMR (400 MHz, CDCl₃) δ 8.81 (s, 1H), 8.15 (s, 1H), 8.09 - 8.02 (m, 2H), 7.68 - 7.59 (m, 1H), 7.55 (t, *J =* 7.6 Hz, 2H), 5.98 (d, *J =* 6.7 Hz, 1H), 4.63 (d, *J=* 6.6 Hz, 2H), 4.25 (d, *J* = 1.6 Hz, 1H), 4.00 (dd, *J* = 13.1, 1.7 Hz, 1H), 3.77 (dd, *J* = 13.1, 1.7 Hz, 1H), 3.30 (s, 3H), 2.98 (s, 1H), 2.90 (s, 1H), 0.99 (s, 9H), 0.19 (s, 3H), 0.17 (s, 3H).

### Step 3: Synthesis of YK-VP-004-PM3

YK-VP-004-PM2 (7.03 g, 14.10 mmol) was dissolved in ultra-dry *N,N*-dimethylformamide (105 mL), and NaH (1.69 g, 70.50 mmol) was added portionwise in an ice-water bath. The mixture was stirred for 10 minutes under a nitrogen atmosphere, followed by stirring at room temperature for 30 minutes. Dimethyl (tosyloxy)methylphosphonate (12.43 g, 42.30 mmol) was added in an ice-water bath, and the reaction was then stirred at room temperature for 17 hours. The reaction was monitored by TLC and LC/MS, and when the starting material disappeared and the target molecular weight was detected, the reaction was stopped. 100 mL of ethyl acetate was added for dilution, which was then washed twice with saturated aqueous ammonium chloride solution (100 mL × 2), followed by two washes with water (100 mL × 2). The combined aqueous phases were back-extracted once with 100 mL of ethyl acetate. The organic phase was dried over anhydrous sodium sulfate, filtered, and concentrated by rotary evaporation to obtain a crude product. The crude product was purified by medium-pressure column chromatography (SiO₂, DCM/MeOH = 100/0 to 96/4) to obtain 4.01 g of a white solid with a yield of 45.7%. MS m/z [M + H]⁺ = 622.2. ¹H NMR (400 MHz, DMSO-*d*₆) δ 11.12 (br, 1H), 8.77 (s, 1H), 8.62 (s, 1H), 8.08 - 8.01 (m, 2H), 7.69 - 7.62 (m, 1H), 7.57 - 7.54 (m, 2H), 6.18 (d, *J=* 5.6 Hz, 1H), 4.65 (t, *J* = 5.2 Hz, 1H), 4.37 (t, *J=* 4.3 Hz, 1H), 4.19 (q, *J=* 4.0 Hz, 1H), 4.10 - 4.04 (m, 2H), 3.90 (dd, *J =* 11.5, 4.2 Hz, 1H), 3.81 (dd, *J=* 11.4, 4.2 Hz, 1H), 3.74 (d, *J=* 1.7 Hz, 3H), 3.72 (d, *J=* 1.7 Hz, 3H), 3.42 (s, 3H), 0.89 (s, 9H), 0.08 (s, 6H).

### Step 4: Synthesis of YK-VP-004-PM4

YK-VP-004-PM3 (4.01 g, 6.45 mmol) was dissolved in 80 mL of ultra-dry tetrahydrofuran, followed by the addition of triethylamine trihydrofluoride (5.25 mL, 32.20 mmol), and the mixture was stirred at 40°C under a nitrogen atmosphere for 16 hours. The reaction was monitored by LC/MS and TLC, and when the starting material disappeared and the target molecular weight was detected, the reaction was stopped. The solvent was removed by rotary evaporation, and the residue was co-evaporated on a rotary evaporator once with acetonitrile to obtain a crude product. The crude product was purified by medium-pressure column chromatography (SiO₂, DCM/MeOH = 100/0 to 94/6) to obtain 1.97 g of a white solid with a yield of 60.2%. MS m/z [M + H]⁺ = 508.1. ¹H NMR (400 MHz, DMSO-*d*₆) δ 11.22 (s, 1H), 8.77 (s, 1H), 8.76 (s, 1H), 8.09 - 8.01 (m, 2H), 7.70 - 7.60 (m, 1H), 7.57 - 7.53 (m, 2H), 6.17 (d, *J=* 6.0 Hz, 1H), 5.30 (t, *J =* 5.5 Hz, 1H), 4.65 (t, *J=* 5.4 Hz, 1H), 4.38 (dd, *J =* 4.7, 3.2 Hz, 1H), 4.19 (q, *J =* 3.7 Hz, 1H), 4.15 - 4.02 (m, 2H), 3.75 - 3.69 (m, 7H), 3.66 - 3.61 (m, 1H), 3.40 (s, 3H). ³¹P NMR (162 MHz, DMSO-*d*₆) δ 23.22.

### Step 5: Synthesis of YK-VP-004

YK-VP-004-PM4 (1.02 g, 2.00 mmol) was co-evaporated on a rotary evaporator with ultra-dry dichloromethane three times. The dried YK-VP-004-PM4 was dissolved in ultra-dry dichloromethane (20 mL), and then 2-cyanoethyl *N,N,N',N'*-tetraisopropylphosphorodiamidite (1.08 g, 1.14 mL, 3.60 mmol) and DCI (354 mg, 3.00 mmol) were added successively. The mixture was stirred at room temperature for 1.5 hours under a nitrogen atmosphere. The reaction was monitored by LC/MS, and when the starting material disappeared and the target molecular weight was detected, the reaction was stopped. The reaction was quenched by adding saturated aqueous sodium bicarbonate solution, and the mixture was extracted with dichloromethane (30 mL × 3). The organic phase was dried over anhydrous sodium sulfate, filtered, and concentrated by rotary evaporation to obtain an oil, which was purified by medium-pressure column chromatography (SiO₂, PE + 1% TEA/EA + 1% TEA = 100/0 to 0/100) to obtain 630 mg of a white solid with a yield of 43.6%. MS m/z [M - H]⁻ = 706.2. ¹H NMR (400 MHz, DMSO-*d*₆) δ 11.22 (s, 1H), 8.76 (s, 1H), 8.65 (d, *J =* 4.3 Hz, 1H), 8.08 - 8.01 (m, 2H), 7.67 - 7.63 (m, 1H), 7.57 - 7.53 (m, 2H), 6.18 (dd, *J* = 5.9, 2.6 Hz, 1H), 4.75 - 4.70 (m, 1H), 4.45 - 4.40 (m, 1H), 4.34 - 4.29 (m, 1H), 4.17 - 3.89 (m, 3H), 3.84 - 3.71 (m, 9H), 3.65 - 3.51 (m, 2H), 3.41 (d, *J* = 2.0 Hz, 3H), 2.81 - 2.77 (m, 2H), 1.16 - 1.10 (m, 12H). ³¹P NMR (162 MHz, DMSO-*d*₆) δ 147.79, 147.63, 23.02, 22.99.

### 5. Synthesis of YK-VP-005

The synthetic route is as follows:

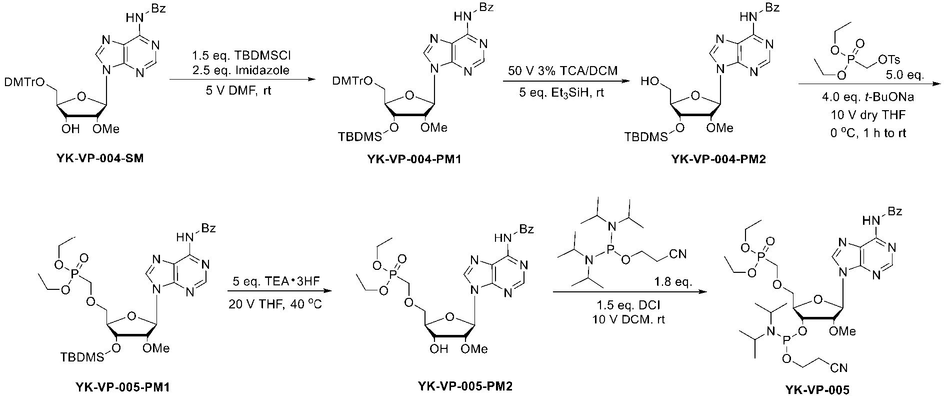

### Step 1: Synthesis of YK-VP-005-PM1

YK-VP-004-PM2 (0.43 g, 0.85 mmol) was dissolved in ultra-dry tetrahydrofuran (5 mL), and sodium *tert*-butoxide (0.33 g, 3.40 mmol) was added in an ice-water bath. The mixture was stirred under a nitrogen atmosphere for 1 hour and then further stirred at room temperature for an additional 1 hour. Diethyl (tosyloxy)methylphosphonate (1.1 mL, 1.37 g, 4.25 mmol) was added, and the reaction was stirred at room temperature overnight. The reaction was monitored by TLC and LC/MS, and when no further conversion of the starting material was observed, the reaction was stopped. 10 mL of dichloromethane was added for dilution, and the reaction was quenched with 10 mL of saturated aqueous ammonium chloride solution. The mixture was extracted and washed with dichloromethane twice (10 mL × 2). The organic phase was dried over anhydrous sodium sulfate, filtered, and concentrated by rotary evaporation to obtain an oil. The oil was purified by medium-pressure column chromatography (SiO₂, DCM/MeOH = 100/0 to 95/5) to obtain 0.19 g of a white solid with a yield of 34.6%. MS m/z [M + H]⁺ = 650.0. ¹H NMR (400 MHz, CDCl₃) δ 9.13 (s, 1H), 8.86 (s, 1H), 8.60 (s, 1H), 8.10 - 8.03 (m, 2H), 7.70 - 7.61 (m, 1H), 7.57 (dd, *J =* 8.4, 6.9 Hz, 2H), 6.28 (d, *J=* 4.0 Hz, 1H), 4.56 (t, *J=* 5.0 Hz, 1H), 4.28 - 4.20 (m, 6H), 4.01 (dd, *J =* 10.7, 3.0 Hz, 1H), 3.91 (d, *J =* 8.3 Hz, 2H), 3.84 (dd, *J =* 10.8, 3.0 Hz, 1H), 3.53 (s, 3H), 1.39 (td, *J =* 7.1, 3.3 Hz, 6H), 0.96 (s, 9H), 0.16 (s, 3H), 0.15 (s, 3H). ³¹P NMR (162 MHz, CDCl₃) δ 20.82.

### Step 2: Synthesis of YK-VP-005-PM2

YK-VP-005-PM1 (0.14 g, 0.21 mmol) was dissolved in 1.8 mL of ultra-dry tetrahydrofuran, and triethylamine trihydrofluoride (0.17 g, 173 µL, 1.06 mmol) was added. The mixture was stirred at 40°C for 16 hours under a nitrogen atmosphere. The reaction was monitored by TLC and LC/MS, and when the starting material disappeared and the target molecular weight was detected, the reaction was stopped. Saturated aqueous sodium bicarbonate solution was slowly added to quench the reaction, and the mixture was extracted with dichloromethane (10 mL × 3). The organic phase was dried over anhydrous sodium sulfate, filtered, and concentrated by rotary evaporation to obtain a crude product. The crude product was purified by medium-pressure column chromatography (SiO₂, DCM/MeOH = 100/0 to 91/9) to obtain 0.09 g of a white solid with a yield of 79.2%. MS m/z [M + H]⁺ = 536.0. ¹H NMR (400 MHz, CDCl₃) δ 9.27 (br, 1H), 8.79 (s, 1H), 8.56 (s, 1H), 8.05 - 8.00 (m, 2H), 7.61 - 7.56 (m, 1H), 7.52 - 7.48 (m, 2H), 6.28 (d, *J =* 3.5 Hz, 1H), 4.52 (t, *J =* 5.3 Hz, 1H), 4.24 - 4.15 (m, 6H), 4.01 (dd, *J=* 10.8, 2.5 Hz, 1H), 3.92 - 3.82 (m, 3H), 3.56 (s, 3H), 2.43 (s, 1H), 1.33 (td, *J* = 7.1, 5.2 Hz, 6H).

### Step 3: Synthesis of YK-VP-005

YK-VP-005-PM2 (0.69 g, 1.30 mmol) was co-evaporated on a rotary evaporator with ultra-dry dichloromethane three times. The dried YK-VP-002-PM2 was dissolved in ultra-dry dichloromethane (14 mL), and then 2-cyanoethyl N,N,N',N'-tetraisopropylphosphorodiamidite (0.69 g, 730 µL, 2.30 mmol) and DCI (0.23 g, 1.95 mmol) were added successively. The mixture was stirred at room temperature for 2.5 hours under a nitrogen atmosphere. The reaction was monitored by LC/MS and TLC, and when the starting material disappeared and the target molecular weight was detected, the reaction was stopped. Saturated aqueous sodium bicarbonate solution was added to quench the reaction, and the mixture was extracted with dichloromethane (20 mL × 3). The organic phase was dried over anhydrous sodium sulfate, filtered, and concentrated by rotary evaporation to obtain a crude product. The crude product was purified by medium-pressure column chromatography (SiO₂, PE + 1% TEA/EA + 1% TEA = 100/0 to 0/100) to obtain 0.70 g of a white solid with a yield of 73.1%. MS m/z [M - H]⁻ = 734.4. ¹H NMR (400 MHz, Acetonitrile-*d*₃) δ 9.47 (s, 1H), 8.70 (d,*J* = 1.8 Hz, 1H), 8.64 (d, *J* = 10.5 Hz, 1H), 8.05 (d, *J =* 7.2 Hz, 2H), 7.68 (t, *J =* 7.4 Hz, 1H), 7.58 (t, *J* = 7.7 Hz, 2H), 6.24 (t, *J =* 5.8 Hz, 1H), 4.77 - 4.65 (m, 1H), 4.56 - 4.46 (m, 1H), 4.44 - 4.34 (m, 1H), 4.24 - 4.09 (m, 4H), 3.95 - 3.87 (m, 4H), 3.84 - 3.67 (m, 3H), 3.49 (s, 1H), 3.43 (s, 1H), 2.74 (t, *J* = 6.0 Hz, 2H), 1.39 - 1.19 (m, 20H). ³¹P NMR (162 MHz, Acetonitrile-*d*₃) δ 150.86, 149.73, 20.83, 20.69.

### 6. Synthesis of Phosphoramidite 3

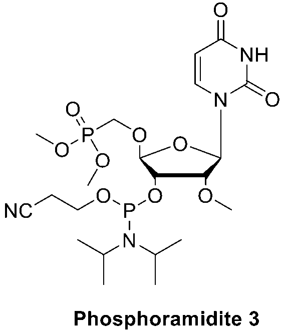

Phosphoramidite 3 was synthesized according to the method described in WO2018045317Al, and 438 mg of product was obtained with an overall yield of 1.9%.

### Example 2: Preparation of siRNA

In this example, two siRNA parent sequences were synthesized, named D84-DV27-G5 and D82-DV29-G5, respectively. Phosphate modification was performed at the last position of D84-DV27-G5 and D82-DV29-G5, respectively, and the resulting double-stranded siRNA sequences are shown in Table 1.

**Table 1: Double-Stranded siRNA Sequences**

| Sequence Number | Sequence Name | SEQ ID NO | Sequence (5' - 3') | |
|---|---|---|---|---|
| 1 | D84-DV27-G5 (Parent Sequence) | 1 | SS | Gms-Ums-Um-Um-Um-Gm-Uf-Am-Gf-Cf-Af-Um- Um-Um-Um-Um-Af-Um-Um-Am-Am-G5 |
| | | 2 | AS | Ums-Ufs-Am-Af-Uf-Af-Am-Am-Am-Am-Um-Gm- Cm-Uf-Am-Cf-Am-Am-Am-Am-Cms-Cms-Cm |
| 2 | D84-DV27-PG5 (Comparative Example) | 3 | SS | Gms-Ums-Um-Um-Um-Gm-Uf-Am-Gf-Cf-Af-Um- Um-Um-Um-Um-Af-Um-Um-Am-Am-G5 |
| | | 4 | AS | [UmsEVP]-Ufs-Am-Af-Uf-Af-Am-Am-Am-Am- Um-Gm-Cm-Uf-Am-Cf-Am-Am-Am-Am-Cms- Cms-Cm |
| 3 | D84-DV27-5EMPG5 | 5 | SS | Gms-Ums-Um-Um-Um-Gm-Uf-Am-Gf-Cf-Af-Um- Um-Um-Um-Um-Af-Um-Um-Am-Am-G5 |
| | | 6 | AS | [Ums-5’-O-EtMP]-Ufs-Am-Af-Uf-Af-Am-Am-Am- Am-Um-Gm-Cm-Uf-Am-Cf-Am-Am-Am-Am-Cms- Cms-Cm |
| 4 | D84-DV27-5MMPG5 | 7 | SS | Gms-Ums-Um-Um-Um-Gm-Uf-Am-Gf-Cf-Af-Um- Um-Um-Um-Um-Af-Um-Um-Am-Am-G5 |
| | | 8 | AS | [Ums-5'-O-MeMP]-Ufs-Am-Af-Uf-Af-Am-Am- Am-Am-Um-Gm-Cm-Uf-Am-Cf-Am-Am-Am-Am- Cms-Cms-Cm |
| 5 | D84-DV27-5MEPG5 | 9 | SS | Gms-Ums-Um-Um-Um-Gm-Uf-Am-Gf-Cf-Af-Um- Um-Um-Um-Um-Af-Um-Um-Am-Am-G5 |
| | | 10 | AS | [Ums-5’-O-MeEP]-Ufs-Am-Af-Uf-Af-Am-Am-Am- Am-Um-Gm-Cm-Uf-Am-Cf-Am-Am-Am-Am-Cms- Cms-Cm |
| 6 | D84-DV27-4MMPG5 (Comparative Example) | 11 | SS | Gms-Ums-Um-Um-Um-Gm-Uf-Am-Gf-Cf-Af-Um- Um-Um-Um-Um-Af-Um-Um-Am-Am-G5 |
| | | 12 | AS | [Ums-4’-O-MeMP]-Ufs-Am-Af-Uf-Af-Am-Am- Am-Am-Um-Gm-Cm-Uf-Am-Cf-Am-Am-Am-Am- Cms-Cms-Cm |
| 7 | D82-DV29-G5 (Parent Sequence) | 13 | SS | Gms-Ams-Am-Gm-Am-Um-Af-Um-Uf-Um-Af- Um-Um-Cm-Um-Gm-Gf-Gm-Um-Um-Um-G5 |
| | | 14 | AS | Ams-Afs-Am-Cf-Cf-Cf-Am-Gm-Am-Am-Um-Am- Am-Af-Um-Af-Um-Cm-Um-Um-Cms-Ams-Am |
| 8 | D82-DV29-5MMPG5 | 15 | SS | Gms-Ams-Am-Gm-Am-Um-Af-Um-Uf-Um-Af- Um-Um-Cm-Um-Gm-Gf-Gm-Um-Um-Um-G5 |
| | | 16 | AS | [Ams-5’-O-MeMP]-Afs-Am-Cf-Cf-Cf-Am-Gm-Am- Am-Um-Am-Am-Af-Um-Af-Um-Cm-Um-Um- Cms-Ams-Am |
| 9 | D82-DV29-5EMPG5 | 17 | SS | Gms-Ams-Am-Gm-Am-Um-Af-Um-Uf-Um-Af- Um-Um-Cm-Um-Gm-Gf-Gm-Um-Um-Um-G5 |
| | | 18 | AS | [Ams-5’-O-EtMP]-Afs-Am-Cf-Cf-Cf-Am-Gm-Am- Am-Um-Am-Am-Af-Um-Af-Um-Cm-Um-Um- Cms-Ams-Am |
| 10 | D82-DV29-5MPG5 | 19 | SS | Gms-Ams-Am-Gm-Am-Um-Af-Um-Uf-Um-Af- Um-Um-Cm-Um-Gm-Gf-Gm-Um-Um-Um-G5 |
| | | 20 | AS | [Ams-5’-O-MP]-Afs-Am-Cf-Cf-Cf-Am-Gm-Am- Am-Um-Am-Am-Af-Um-Af-Um-Cm-Um-Um- Cms-Ams-Am |

In this text, the meanings of the abbreviations are as follows:
A, U, G, and C represent natural adenosine ribonucleotide, uridine ribonucleotide, guanosine ribonucleotide, and cytidine ribonucleotide, respectively.

m indicates that the nucleotide adjacent to it on the left is a nucleotide modified with 2'-OMe (2'-methoxy nucleoside). For example, Am, Um, Gm, and Cm represent 2'-OMe-modified A, U, G, and C, respectively.

f indicates that the nucleotide adjacent to it on the left is a nucleotide modified with 2'-F (2'-fluoro nucleoside). For example, Af, Uf, Gf, and Cf represent 2'-F-modified A, U, G, and C, respectively.

s indicates that the two nucleotides adjacent to it on the left and right and/or the delivery support are connected via a phosphorothioate linkage.

EVP indicates that the nucleotide adjacent to it on the left is a nucleotide modified with 5'-(E)-VP, and the structure of UmEVP is shown in Table 2.

5'-O-EtMP indicates that the nucleoside structure in the nucleotide adjacent to it on the left is provided by YK-VP-001, and the structure of Ums-5'-O-EtMP is shown in Table 2.

5'-O-MeMP indicates that the nucleoside structure in the nucleotide adjacent to it on the left is provided by YK-VP-002, and the structure of Ums-5'-O-MeMP is shown in Table 2.

5'-O-MeEP indicates that the nucleoside structure in the nucleotide adjacent to it on the left is provided by YK-VP-003, and the structure of Ums-5'-O-MeEP is shown in Table 2.

4'-O-MeMP indicates that the nucleoside structure in the nucleotide adjacent to it on the left is provided by Phosphoramidite 3, and the structure of Ums-4'-O-MeMP is shown in Table 2.

5'-O-MeMP indicates that the nucleoside structure in the nucleotide adjacent to it on the left is provided by YK-VP-004, and the structure of Am-5'-O-MeMP is shown in Table 2.

5'-O-EtMP indicates that the nucleoside structure in the nucleotide adjacent to it on the left is provided by YK-VP-005, and the structure of Am-5'-O-EtMP is shown in Table 2.

5'-O-MP indicates that the nucleoside structure in the nucleotide adjacent to it on the left is provided by YK-VP-004, and the structure of Am-5'-O-MP is shown in Table 2.

G5 represents a GalNAc delivery support having the following structure, wherein 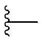 represents linkage to the 3' end of the sense strand of the siRNA via a phosphate group or a thiophosphate group, as described in CN116854754A:

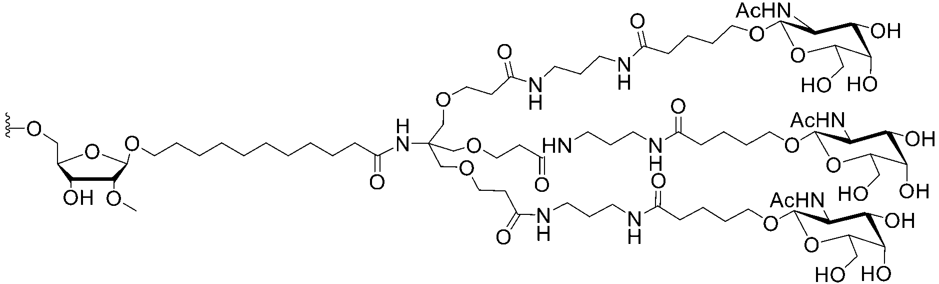

### 1. Preparation of siRNA antisense strand without GalNAc conjugation

The siRNA antisense strand was synthesized on the corresponding solid-phase support using the phosphoramidite chemistry.

In the synthesis of sequence 2 (D84-DV27-PG5), a commercially purchased (E)-VP-Um monomer was used as the last coupling monomer of the antisense strand. In the synthesis of sequences 3 to 5 (D84-DV27-5EMPG5, D84-DV27-5MMPG5, and D84-DV27-5MEPG5), the 5'-phosphate modified nucleoside phosphoramidite monomers (YK-VP-001, YK-VP-002, and YK-VP-003) synthesized in Example 1 were respectively used as the last coupling monomer of the antisense strand. In the synthesis of sequence 6 (D84-DV27-4MMPG5), Phosphoramidite 3 monomer from Example 1 was used as the last coupling monomer of the antisense strand.

In the synthesis of sequences 8 to 10 (D82-DV29-5MMPG5, D82-DV29-5EMPG5, and D82-DV29-5MPG5), the 5'-phosphate modified nucleoside phosphoramidite monomers synthesized in Example 1 (YK-VP-004 and YK-VP-005) were respectively used as the last coupling monomer of the antisense strand, and the synthesis scale was 200 nmol for all of them.

The end structures of the antisense strands of the synthesized siRNAs are shown in Table 2.

**Table 2: End Structures of Antisense Strands**

| Name | Monomer Structure | Source | 5' End Structure in Antisense Strand |
|---|---|---|---|
| YK-VP-001 | 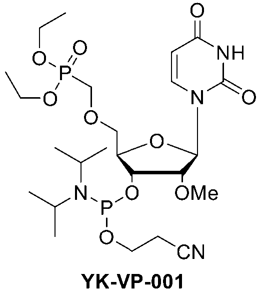 | Designed in the Present Disclosure, Synthesized in Example 1 | 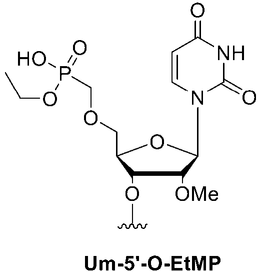 |
| YK-VP-002 | 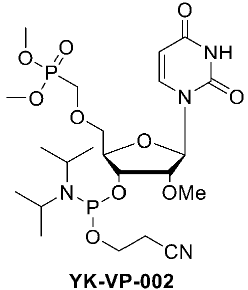 | Designed in the Present Disclosure, Synthesized in Example 1 | 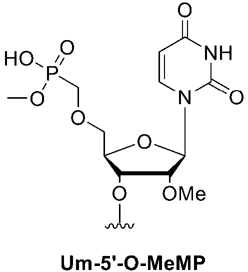 |
| YK-VP-003 | 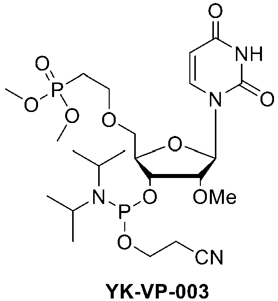 | Designed in the Present Disclosure, Synthesized in Example 1 | 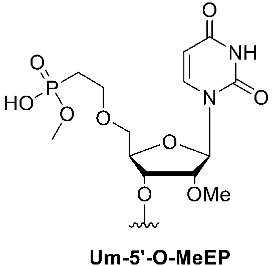 |
| YK-VP-004 | 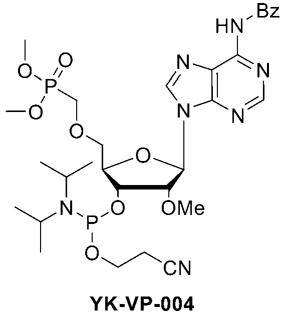 | Designed in the Present Disclosure, Synthesized in Example 1 | 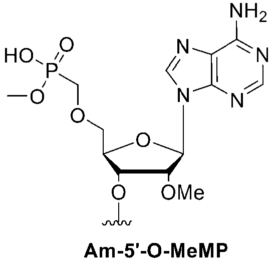 |
| YK-VP-005 | 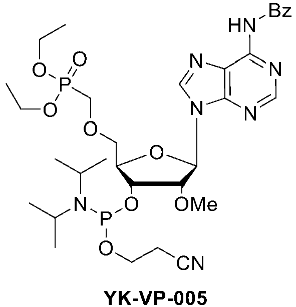 | Designed in the Present Disclosure, Synthesized in Example 1 | 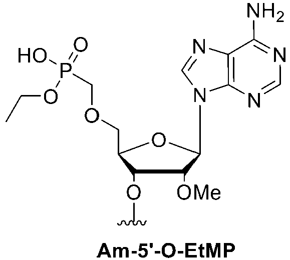 |
| YK-VP-004 | 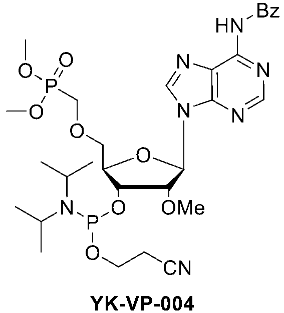 | Designed in the Present Disclosure, Synthesized in Example 1 | 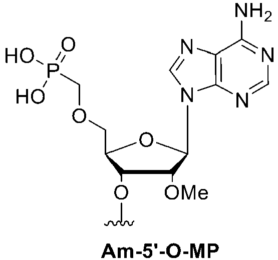 |
| Reference (E)-VP-Um | 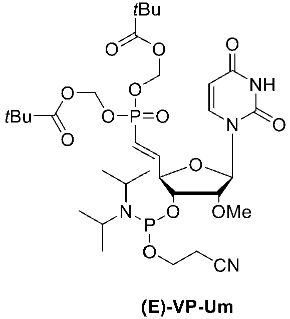 | Purchased from Qingdao Glycogene Pharmaceutical Technology Co., Ltd. | 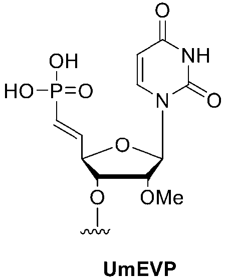 |
| Reference Phosphoramidite 3 | 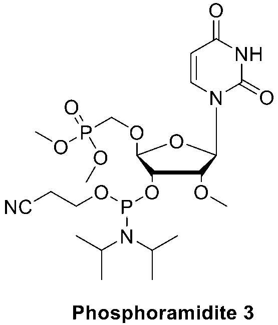 | Synthesized in Example 1 of WO2018045317 A1 | 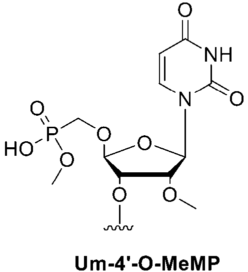 |

### (1) Preparation of reagents and monomers

An acetonitrile solution of the monomer (0.15 M), an acetonitrile solution of 0.25 M 5-(ethylthio)tetrazole as an activator, a pyridine solution of 3% xanthane hydride as a sulfurizing agent, a water/pyridine (10/90, v/v) solution of 0.05 M iodine as an oxidizing reagent, an acetonitrile solution of 10% acetic anhydride (v/v) as capping reagent A, a 1-methylimidazole/pyridine/tetrahydrofuran solution (16/10/74, v/v/v) as capping reagent B, and a toluene solution of 3% dichloroacetic acid (v/v) as a de-DMTr reagent were used. A universal crosslinked polystyrene bead support (Primer support 5G Unylinker 350, manufactured by Cytiva) was selected as the solid-phase support, and each was loaded into the specified reagent positions of a 192 P model DNA/RNA automatic synthesizer.

### (2) Crude product synthesis

The specified oligonucleotide sequence was entered, and the synthesis program was set. After verification, cyclic oligonucleotide synthesis was initiated. The oligonucleotide was prepared according to the following steps:

### a. Deprotection

A toluene solution of 3% dichloroacetic acid was used as the deprotection reagent to remove the DMTr protecting group, followed by rinsing with acetonitrile.

### b. Coupling

A solution of 0.25 M 5-(ethylthio)tetrazole was used as the activator to couple the acetonitrile solutions of each nucleotide monomer, followed by rinsing with acetonitrile.

### c. Oxidation/sulfurization

Oxidation: An aqueous/pyridine (90/10) solution of 0.05 M iodine was used as the oxidizing agent for oxidation, followed by rinsing with acetonitrile.

Sulfurization: A pyridine solution of 3% xanthane hydride was used as the sulfurizing agent for sulfurization, followed by rinsing with acetonitrile.

### d. Hydroxyl protection

Capping reagent A and capping reagent B were used as hydroxyl protection reagents to carry out hydroxyl protection, followed by rinsing with acetonitrile.

The above operations were repeated according to the set sequence cycles to obtain fully protected products.

### (3) Deprotection

### a. Preparation of monomethyl ester-protected 5'-phosphate modified siRNA antisense strand

The solid-phase support was transferred into a reactor, concentrated ammonia water (25-28%) was added, and after ammonolysis was maintained at 60°C for 12 hours, the system was cooled to room temperature, and the mixture was transferred into a pressure filtration vessel. The mixture was rinsed with a mixed solution of purified water and ethanol, and the filtrates were combined and concentrated at low temperature to obtain the crude product of monomethyl ester-protected 5'-phosphate modified siRNA antisense strand.

### b. Preparation of fully deprotected 5'-phosphate modified siRNA antisense strand

The solid-phase support was transferred into a reactor, and a TMSI/Py/DCM solution was added. After reaction at room temperature for 1 hour, a TEA/ACN solution of 2-mercaptoethanol was added to quench the reaction. Subsequent treatment was then carried out according to deprotection method a, and the crude product of fully deprotected 5'-phosphate modified siRNA antisense strand was obtained.

### (4) Purification

The deprotected crude residue was dissolved in purified water and purified by HPLC. The product peak fraction was collected, its content was determined using a microplate reader, and its purity and molecular weight were confirmed by LC/MS. The product peak fraction was concentrated and lyophilized to obtain the final product.

### 2. Preparation of GalNAc-conjugated siRNA sense strand

The siRNA sense strand was synthesized according to the method used for synthesizing the siRNA antisense strand. Among them, the G5-GalNAc support was used as a solid-phase support, and each sense strand complementary to the antisense strand was synthesized on a 200 nmol scale.

### 3. Preparation of double-stranded siRNA

The siRNA sense strand and complementary antisense strand were mixed at a 1:1 ratio based on UV absorbance, heated to 95°C, maintained for 3 minutes, and then slowly cooled to room temperature to form double strands. The resulting double-stranded solution was characterized by HPLC to confirm that the product purity was acceptable, and its content was measured using a microplate reader. The solution was then lyophilized to obtain a solid powder for storage. The molecular weight and purity of the resulting double-stranded siRNA are shown in Table 3.

**Table 3: Molecular Weight and Purity of Double-Stranded siRNA**

| Sequence Number | Sequence Name | Sequence Direction (5' - 3') | Theoretical Molecular Weight | Measured Molecular Weight | Purity (%) |
|---|---|---|---|---|---|
| 1 | D84-DV27-G5 (Parent Sequence) | SS | 8671.79 | 8671.44 | 97.94 |
| | | AS | 7604.34 | 7603.20 | |
| 2 | D84-DV27-PG5 (Comparative Example) | SS | 8671.79 | 8671.44 | 97.09 |
| | | AS | 7680.33 | 7679.17 | |
| 3 | D84-DV27-5EMPG5 | SS | 8671.79 | 8671.44 | 92.44 |
| | | AS | 7727.14 | 7724.65 | |
| 4 | D84-DV27-5MMPG5 | SS | 8671.79 | 8671.44 | 97.03 |
| | | AS | 7713.17 | 7711.16 | |
| 5 | D84-DV27-5MEPG5 | SS | 8671.79 | 8671.44 | 96.66 |
| | | AS | 7727.26 | 7725.30 | |
| 6 | D84-DV27-4MMPG5 (Comparative Example) | SS | 8671.79 | 8671.44 | 94.83 |
| | | AS | 7699.15 | 7697.08 | |
| 7 | D82-DV29-G5 (Parent Sequence) | SS | 8761.90 | 8761.68 | 95.07 |
| | | AS | 7604.34 | 7603.40 | |
| 8 | D82-DV29-5MMPG5 | SS | 8761.90 | 8761.68 | 97.25 |
| | | AS | 7713.23 | 7711.41 | |
| 9 | D82-DV29-5EMPG5 | SS | 8761.90 | 8761.68 | 95.86 |
| | | AS | 7727.13 | 7725.20 | |
| 10 | D82-DV29-5MPG5 | SS | 8761.90 | 8761.68 | 96.53 |
| | | AS | 7699.31 | 7697.49 | |

**Example 3: Inhibitory effect of modified oligonucleotide sequences on PCSK9 and their**

### impact on LDL-C levels in mouse serum

In this example, the inhibitory rate of PCSK9 and the impact on LDL-C levels in mouse serum by the siRNA sequences in Table 1 were investigated.

### Experimental materials

Test drug:
Modified siRNA sequences listed in Table 1
Preparation of test drug:
Drug vehicle: PBS buffer
Preparation conditions: Sterile environment
Labeling method: The prepared dosing formulations were labeled, and the outer packaging was marked with the subject number, name, concentration, quantity, preparation date, preparer, and storage conditions;
Storage conditions: Prepared freshly before use; remaining samples were stored at -20°C.
Experimental animal information:
Species/strain: B6-hPCSK9-UTR (T053388) mice
Grade: SPF
Gender: Male
Quantity: 59
Age: 6-8 weeks
Source: GemPharmatech Co., Ltd., Jiangsu
Feeding and management:
   Feeding conditions: After receipt, the experimental animals were fed at GemPharmatech Co., Ltd., Jiangsu, with free access to food and water. Standard SPF-grade irradiated and sterilized experimental transgenic mouse feed was used, which was purchased from Jiangsu Xietong Pharmaceutical Bio-Engineering Co., Ltd. For each batch of feed, a certificate of feed quality conformity was provided by the manufacturer, and a third-party test report was provided annually; the test standards referred to national standards GB 14924.3-2010 "Laboratory animals - Nutrients for formula feeds" and GB 14924.2-2001 "Laboratory animals - Hygienic standard for formula feeds". The appearance and bacterial indicators of drinking water were tested once a month within the company, and an annual test report was provided by the municipal water supply company in this district, with reference to the national standard GB5749-2006 "Standards for Drinking Water Quality".

### 1. Inhibitory rate of siRNA sequences with different modifications on PCSK9 protein expression in mouse serum

### Experimental methods:

The day of administration was designated as D0, and approximately 200 µL of blood was collected from the medial canthus of the eye on D-3. Whole blood samples were temporarily stored in an ice box before centrifugation, then centrifuged for 10 min at approximately 4°C and a centrifugal force of approximately 3000 g. PCSK9 levels were measured using a PCSK9 kit (purchased from Proteintech), and the animals were grouped accordingly, with 6 mice in each group, so as to ensure that the PCSK9 level of mice in each group was uniform. On D0 (Day 0), the drug was administered by subcutaneous injection, at a dose of 2 mg/kg. On D7 (Day 7), D14 (Day 14), and D21 (Day 21), blood was collected from the medial canthus of the eye, serum was separated, and the PCSK9 protein level was measured.

### Experimental results:

The inhibitory rates of PCSK9 protein in serum on Days 7, 14, and 21 of administration are shown in Table 4.

**Table 4: Inhibitory Rate of PCSK9 Protein Expression in Mouse Serum**

| Sequence Name | Inhibitory Rate (%) | | |
|---|---|---|---|
| | Day 7 | Day 14 | Day 21 |
| D84-DV27-G5 (Parent Sequence) | 71.8 | 72.5 | 64.2 |
| D84-DV27-PG5 (Comparative Example) | 76.6 | 83.3 | 74.1 |
| D84-DV27-5EMPG5 | 85.2 | 88.6 | 79.4 |
| D84-DV27-5MMPG5 | 88.3 | 90.6 | 83.9 |
| D84-DV27-5MEPG5 | 87.9 | 89.2 | 80.5 |
| D84-DV27-4MMPG5 (Comparative Example) | 76.5 | 74.1 | 68.6 |
| D82-DV29-G5 (Parent Sequence) | 73.4 | 75.7 | 70.2 |
| D82-DV29-5MMPG5 | 80.6 | 87.1 | 81.8 |
| D82-DV29-5EMPG5 | 82.0 | 89.3 | 83.5 |
| D82-DV29-5MPG5 | 78.4 | 80.5 | 72.7 |

### 1) The 5'-phosphate modified siRNA sequences of the present disclosure are all capable of efficiently inhibiting the expression of PCSK9 protein in mouse serum.

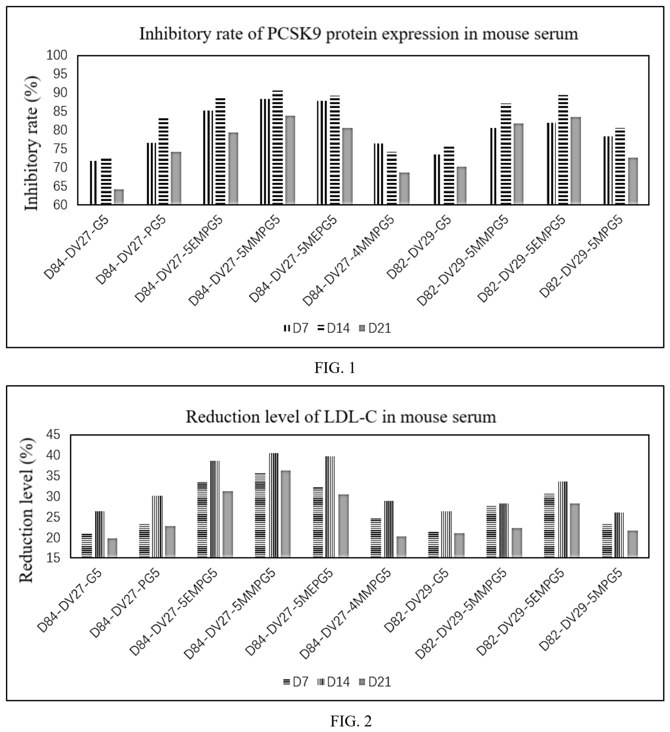

It can be seen from Table 4 and FIG. 1 that the 5'-phosphate modified siRNA sequences according to the present disclosure exhibit a significant inhibitory effect on PCSK9 protein expression in mouse serum. For example, the inhibitory rates of D84-DV27-5EMPG5, D84-DV27-5MMPG5, and D84-DV27-5MEPG5 on Day 7, Day 14, and Day 21 are all higher than that of D84-DV27-G5. Among them, the inhibitory rates of D84-DV27-5MMPG5 modified with YK-VP-002 on Day 7, Day 14, and Day 21 are 16.5%, 18.1%, and 19.7% higher than that of D84-DV27-G5, respectively.

For example, the inhibitory rates of D82-DV29-5MMPG5 and D82-DV29-5EMPG5 on Day 7, Day 14, and Day 21 are both higher than that of D82-DV29-G5. Among them, the inhibitory rates of D82-DV29-5EMPG5 modified with YK-VP-005 on Day 7, Day 14, and Day 21 are 8.6%, 13.6%, and 13.3% higher than that of D82-DV29-G5, respectively.

### 2) Compared with siRNA sequences modified with compounds in the prior art, the 5'-phosphate modified siRNA sequences according to the present disclosure are found to significantly increase the inhibitory rate of PCSK9 protein expression in mouse serum.

The inhibitory rates on Day 7, Day 14, and Day 21 of siRNA sequences modified with compounds in the prior art, including D84-DV27-PG5 modified with (E)-VP-Um and D84-DV27-4MMPG5 modified with Phosphoramidite 3, are all lower than those of the 5'-phosphate modified siRNA sequences of the present disclosure. For example, compared with D84-DV27-PG5, the inhibitory rates of D84-DV27-5MMPG5 on Day 7, Day 14, and Day 21 are increased by 11.7%, 7.3%, and 9.8%, respectively. Compared with D84-DV27-4MMPG5, the inhibitory rates of D84-DV27-5MMPG5 on Day 7, Day 14, and Day 21 are increased by 11.8%, 16.5%, and 15.3%, respectively.

Structurally, compared with YK-VP-002, Phosphoramidite 3 lacks one CH₂ group at the 5'-position of the ribose ring, while all other structures are the same; however, the corresponding modified siRNA exhibits a significantly different inhibitory rate on PCSK9 protein expression in mouse serum. Thus, an unexpected effect is achieved by the 5'-phosphate modified siRNA sequence of the present disclosure.

### 2. Effects of siRNA sequences with different modifications on LDL-C levels in mouse serum

### Experimental procedure:

The day of administration was designated as D0 (Day 0), and approximately 200 µL of blood was collected from the medial canthus of the eye on D3 (Day 3). Whole blood samples were temporarily stored in an ice box before centrifugation, then centrifuged for 10 min at approximately 4°C and a centrifugal force of approximately 3000 g. LDL-C levels were measured using a biochemical analyzer, and the animals were grouped accordingly, with 6 mice in each group, so as to ensure that the LDL-C level of mice in each group was uniform. On D0 (Day 0), the drug was administered by subcutaneous injection, at a dose of 2 mg/kg. On D7 (Day 7), D14 (Day 14), and D21 (Day 21), blood was collected from the medial canthus of the eye, serum was separated, and the LDL-C level in serum was measured.

### Experimental results:

On Day 7, Day 14, and Day 21 after administration, the reduction levels of LDL-C in serum were observed, and the specific results are shown in Table 5.

**Table 5: Reduction Levels of LDL-C in Mouse Serum**

| Sequence Name | Reduction Level (%) | | |
|---|---|---|---|
| | Day 7 | Day 14 | Day 21 |
| D84-DV27-G5 (Parent Sequence) | 20.8 | 26.3 | 19.7 |
| D84-DV27-PG5 (Comparative Example) | 23.5 | 30.2 | 22.8 |
| D84-DV27-5EMPG5 | 33.4 | 38.7 | 31.2 |
| D84-DV27-5MMPG5 | 35.8 | 40.5 | 36.3 |
| D84-DV27-5MEPG5 | 32.2 | 39.7 | 30.5 |
| D84-DV27-4MMPG5 (Comparative Example) | 24.6 | 28.9 | 20.3 |
| D82-DV29-G5 (Parent Sequence) | 21.5 | 26.4 | 21.0 |
| D82-DV29-5MMPG5 | 27.6 | 28.3 | 22.3 |
| D82-DV29-5EMPG5 | 30.8 | 33.6 | 28.2 |
| D82-DV29-5MPG5 | 23.6 | 26.1 | 21.7 |

### 1) The 5'-phosphate modified siRNA sequences of the present disclosure are all capable of efficiently reducing LDL-C levels in mouse serum.

It can be seen from Table 5 and FIG. 2 that the 5'-phosphate modified siRNA sequences of the present disclosure are capable of significantly reducing LDL-C levels in mouse serum. For example, on Day 7, Day 14, and Day 21, D84-DV27-5MMPG5 modified with YK-VP-002 is found to reduce the level by 35.8%, 40.5%, and 36.3%, respectively, which are increases of 15.0%, 14.2%, and 16.6%, respectively, compared with the parent sequence D84-DV27-G5.

For example, on Day 7, Day 14, and Day 21, D82-DV29-5EMPG5 modified with YK-VP-005 is found to reduce the level by 30.8%, 33.6%, and 28.2%, respectively, which is significantly higher than that of the parent sequence D82-DV29-G5.

### 2) Compared with siRNA sequences modified by compounds in the prior art, the 5'-phosphate modified siRNA sequences of the present disclosure are found to significantly improve reduction levels of LDL-C in mouse serum.

For siRNA sequences modified with compounds in the prior art, the reduction levels of D84-DV27-PG5 and D84-DV27-4MMPG5 on Day 7, Day 14, and Day 21 are in the range of 20%-31%, all of which are lower than the 30%-41% achieved by the 5'-phosphate modified siRNA sequence of the present disclosure. For example, compared with D84-DV27-PG5, the reduction levels of D84-DV27-5EMPG5 on Day 7, Day 14, and Day 21 are increased by 9.9%, 8.5%, and 8.4%, respectively. Compared with D84-DV27-4MMPG5, the reduction levels of D84-DV27-5MMPG5 on Day 7, Day 14, and Day 21 are increased by 11.2%, 11.6%, and 16.0%, respectively.

From the above experimental results, it can be seen that, compared with siRNA modified with phosphate of the prior art, the 5'-phosphate modified siRNA of the present disclosure has a better effect in reducing LDL-C levels in mouse serum and achieves unexpected effects.

## Claims

1. A modified nucleoside compound, **characterized in that** the modified nucleoside compound is a compound represented by formula (I), a pharmaceutically acceptable salt thereof, or a stereoisomer thereof: 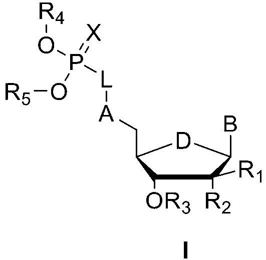 wherein
R₁ is H, and R₂ is -OCH₃;
R₃ is a protecting group or a phosphorus-containing active reactive group, wherein the phosphorus-containing active reactive group is any one of a phosphoramidite, an H-phosphonate, a phosphotriester, or a phosphorus-containing chiral auxiliary, and the phosphorus-containing chiral auxiliary is selected from 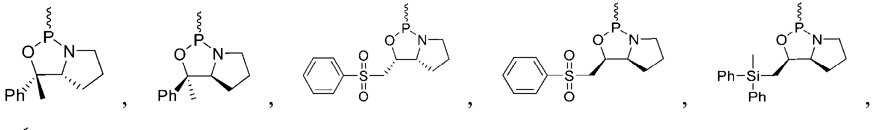 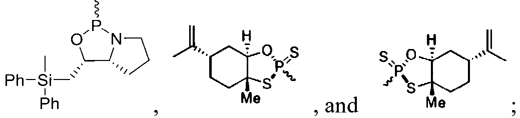
R₄ and R₅ are each independently selected from H, C₁₋₆ alkyl, -CH₂CH₂CN, and - CH₂O(CO)C(CH₃)₃;
X is O or S;
L is -CH₂- or -CH₂CH₂-;
A is O;
B is a modified or unmodified base or a salt thereof;
D is O.

2. The modified nucleoside compound according to claim 1, **characterized in that** R₄ and R₅ are not simultaneously isopropyl; or, R₄ and R₅ are each independently selected from -CH₂CH₃, -CH₃, and H;
or, R₃ is

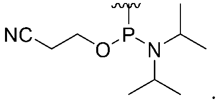

3. The modified nucleoside compound according to claim 1, **characterized in that** R₄ is -CH₃ or - CH₂CH₃;
and/or, R₅ is -CH₃ or -CH₂CH₃; preferably, R₄ and R₅ are the same;
and/or, X is O;
and/or, B is

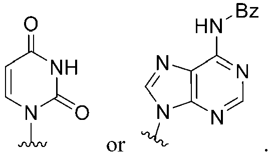

4. The modified nucleoside compound according to claim 1, **characterized in that** the compound of formula (I) is any one of the following compounds YK-VP-001, YK-VP-002, YK-VP-003, YK-VP-004, and YK-VP-005:

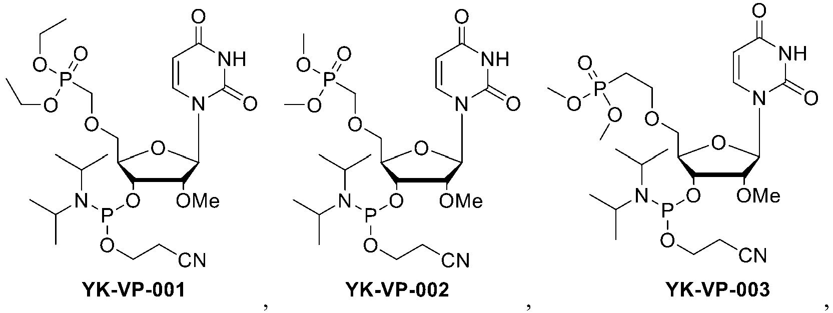

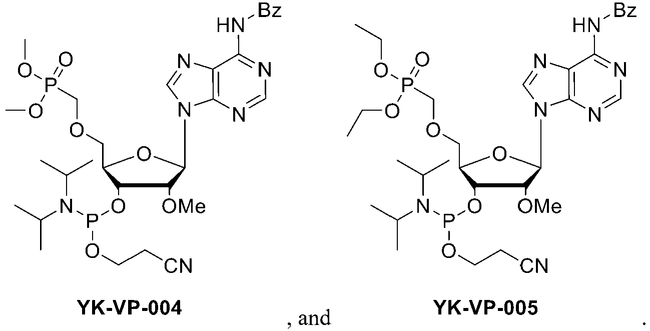

5. An oligonucleotide, **characterized in that** the structural moiety of the oligonucleotide comprises a nucleotide comprising the modified nucleoside compound according to any one of claims 1-4.

6. The oligonucleotide according to claim 5, **characterized in that** the oligonucleotide is an oligonucleotide represented by formula (II) or a pharmaceutically acceptable salt thereof, 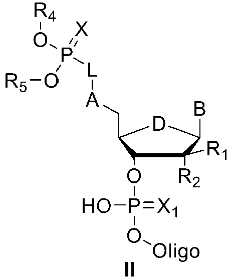 wherein
Oligo is an oligonucleotide,
R₁ is H, and R₂ is -OCH₃;
R₄ and R₅ are each independently selected from H, C₁₋₆ alkyl, -CH₂CH₂CN, and - CH₂O(CO)C(CH₃)₃;
X is O or S;
L is -CH₂- or -CH₂CH₂-;
A is O;
D is O;
X₁ is O or S, and B is

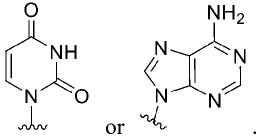

7. The oligonucleotide according to claim 5, **characterized in that** the nucleotide comprising the modified nucleoside compound is located at the 5' end of the oligonucleotide;
or, the oligonucleotide is selected from an siRNA, an antisense oligonucleotide, a microRNA, a small activating RNA, a small guide RNA, a transfer RNA, and an aptamer, preferably an siRNA; preferably, the siRNA is a double-stranded siRNA or a single-stranded siRNA; more preferably, the siRNA is a double-stranded siRNA comprising a sense strand and an antisense strand; further more preferably, the antisense strand in the double-stranded siRNA is the oligonucleotide represented by formula (II) according to claim 6 or a pharmaceutically acceptable salt thereof.

8. The oligonucleotide according to any one of claims 5-7, **characterized in that** each nucleotide in the oligonucleotide is independently a modified or unmodified nucleotide.

9. A nucleic acid conjugate, **characterized in that** the nucleic acid conjugate comprises the oligonucleotide according to any one of claims 5-8 or a pharmaceutically acceptable salt thereof, and a targeting ligand conjugated thereto.

10. The nucleic acid conjugate according to claim 9, **characterized in that** the targeting ligand comprises any one of a carbohydrate, a lipid, a polypeptide, or an antibody;
preferably, the lipid comprises cholesterol;
more preferably, the targeting ligand comprises an N-acetylgalactosamine moiety;
further more preferably, the N-acetylgalactosamine moiety is a monovalent N-acetylgalactosamine moiety, a divalent N-acetylgalactosamine moiety, a trivalent N-acetylgalactosamine moiety, or a tetravalent N-acetylgalactosamine moiety.

11. A pharmaceutical composition, **characterized in that** the pharmaceutical composition comprises the oligonucleotide according to any one of claims 5-8 or a pharmaceutically acceptable salt thereof;
and/or, the pharmaceutical composition comprises the nucleic acid conjugate according to claim 9 or 10.

12. The pharmaceutical composition according to claim 11, **characterized in that** the pharmaceutical composition further comprises a pharmaceutically acceptable excipient.

13. The oligonucleotide according to any one of claims 5-8 or a pharmaceutically acceptable salt thereof, the nucleic acid conjugate according to claim 9 or 10, or the pharmaceutical composition according to claim 11 or 12 for use in treating and/or preventing a pathological condition or disease caused by expression of the proprotein convertase subtilisin/kexin type 9 gene in hepatocytes.

14. The oligonucleotide or the pharmaceutically acceptable salt thereof, the nucleic acid conjugate, or the pharmaceutical composition for use according to claim 13, **characterized in that** the pathological condition or disease is selected from any one or a combination of at least two of dyslipidemia and cardiovascular and cerebrovascular diseases; preferably, the dyslipidemia comprises hypercholesterolemia; and/or, the cardiovascular and cerebrovascular diseases comprise atherosclerosis.

15. A kit, **characterized in that** the kit comprises the oligonucleotide according to any one of claims 5-8 or a pharmaceutically acceptable salt thereof, or the nucleic acid conjugate according to claim 9 or 10, or the pharmaceutical composition according to claim 11 or 12.
